# EUROPEAN PATENT APPLICATION

(11) **EP 2 763 248 A1**
(43) Date of publication of application: **06.08.2014**
(21) Application number: 12835594.8
(22) Date of filing: 14.09.2012
(51) Int. Cl.: H01S 3/121, A61B 8/00, G01N 29/00, H01S 3/123

(54) **LASER SOURCE UNIT AND OPTICAL ACOUSTIC IMAGE GENERATION DEVICE**

(30) Priority: 27.09.2011 JP 2011210143; 10.09.2012 JP 2012198099
(71) Applicant: Fujifilm Corporation, Minato-ku Tokyo 106-8620 (JP)
(72) Inventor: KASAMATSU Tadashi, Kanagawa 258-8538 (JP); HIROTA Kazuhiro, Kanagawa 258-8538 (JP)
(74) Representative: Klunker . Schmitt-Nilson . Hirsch
(86) International application number: PCT/JP2012/073733
(87) International publication number: WO 2013/047250

(57) **Abstract**

A laser source unit obtains Q switch pulse oscillation with a simple structure while continuously switching a plurality of wavelengths. A laser source unit emits pulsed laser beams with a plurality of different wavelengths. A flash lamp radiates excitation light to a laser rod. A pair of mirrors face each other with the laser rod interposed therebetween. The pair of mirrors form an optical resonator. Wavelength selection means controls the wavelength of light which resonates in the optical resonator to any one of a plurality of wavelengths to be emitted by the laser source unit. Driving means drives the wavelength selection means such that the optical resonator performs the Q switch pulse oscillation.

## Description

### BACKGROUND OF THE INVENTION

### 1. Field of the Invention

The present invention relates to a laser source unit, and more particularly, to a laser source unit that switches laser beams with a plurality of wavelengths and emits the laser beams.

In addition, the present invention relates to a photoacoustic image generation apparatus that generates a photoacoustic image based on a photoacoustic signal which is detected for each wavelength when laser beams with a plurality of wavelengths are radiated to a subject.

### 2. Description of the Related Art

For example, JP2005-21380A or A High-Speed Photoacoustic Tomography System based on a Commercial Ultrasound and a Custom Transducer Array, Xueding Wang, Jonathan Cannata, Derek De Busschere, Changhong Hu, J. Brian Fowlkes, and Paul Carson, Proc. SPIE Vol. 7564, 756424 (Feb. 23, 2010) discloses a photoacoustic imaging apparatus that captures the internal image of a living body using a photoacoustic effect. In the photoacoustic imaging apparatus, for example, pulsed light, such as a pulsed laser beam, is radiated to the living body. In the living body irradiated with the pulsed light, the volume of a body tissue which absorbs the energy of the pulsed light is expanded by heat and acoustic waves are generated. For example, an ultrasonic probe can detect the acoustic waves and the internal image of the living body can be formed based on the detected signal (photoacoustic signal). In a photoacoustic imaging method, since the acoustic waves are generated by a specific light absorber, it is possible to capture the image of a specific tissue, for example, a blood vessel in the living body.

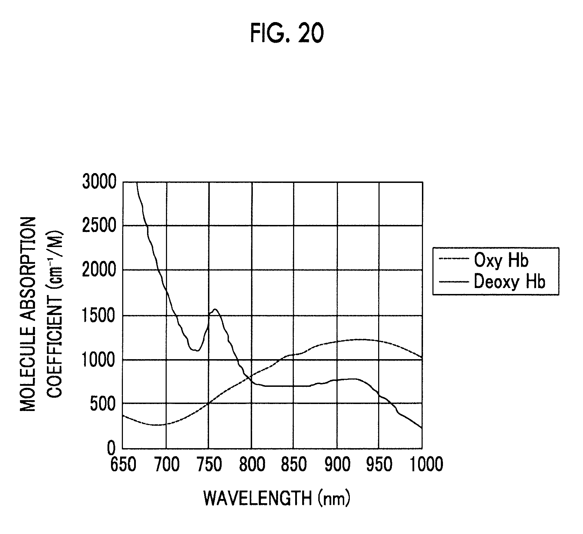

However, the light absorption characteristics of most body tissues vary depending on the wavelength of light. In addition, in general, each tissue has unique light absorption characteristics. For example, Fig. 20 shows the molecular absorption coefficients of oxygenated hemoglobin (hemoglobin combined with oxygen: oxy-Hb) which is contained in large quantities in human arteries, and deoxygenated hemoglobin (hemoglobin which is not combined with oxygen: deoxy-Hb) which is contained in large quantities in veins, for each wavelength of light. The light absorption characteristics of arteries correspond to those of the oxygenated hemoglobin and the light absorption characteristics of veins correspond to those of the deoxygenated hemoglobin. A photoacoustic imaging method has been used which uses the difference in the light absorption rate depending on the wavelength, radiates light components with two different types of wavelengths to blood vessels, and distinctively creates the images of arteries and veins (for example, see JP2010-046215A).

For example, JP1998-65260A (JP-H10-65260A) or JP1998-65238A (JP-H10-65238A) discloses a laser device which radiates laser beams with a plurality of wavelengths. In JP1998-65260A (JP-H10-65260A), a filter which selectively transmits only light with a specific peak wavelength is arranged on an optical path between a laser active medium and one of optical resonator mirrors. Filters corresponding to the number of peak wavelengths to be selected are prepared and any one of the prepared filters is arranged on the optical path, which makes it possible to switch the laser beams with a plurality of wavelengths and to emit the laser beams. JP1998-65238A (JP-H10-65238A) discloses a technique in which one of the mirrors forming the optical resonator has characteristics that selectively reflect only light with a specific peak wavelength. The mirrors having the above-mentioned characteristics are prepared so as to correspond to the number of peak wavelengths to be selected and any one of the prepared mirrors is used to form the optical resonator, which makes it possible to switch the laser beams with a plurality of wavelengths and to emit the laser beams.

In the photoacoustic field, in general, a pulsed laser beam is radiated to the subject. A Q switch method has been known as a technique for generating pulsed laser beams. A mechanical Q switch which generates Q switch oscillation using a mechanical motion has been known as a type of Q switch. For example, the following Q switches have been known as the mechanical Q switch: a Q switch which mechanically controls the gain using a rotating mirror to perform the Q switch oscillation; and a Q switch which rotates a mechanical chopper having a slit or an opening provided therein to adjust the gain. For example, JP2007-235063A discloses a Q switch laser which uses a rotating mechanical chopper.

### SUMMARY OF THE INVENTION

The laser device disclosed in JP1998-65260A (JP-H10-65260A) can switch a plurality of wavelengths. However, JP1998-65260A (JP-H10-65260A) discloses only the structure which switches the filter to be inserted onto the optical path to switch the wavelengths of the laser beams, but does not provide means for continuously switching the laser beams with a plurality of wavelengths and emitting the laser beams. Similarly, JP1998-65238A (JP-H10-65238A) discloses only the structure which switches one of the mirrors forming the optical resonator to switch the wavelength of the laser beam, but does not provide means continuously switching the laser beams with a plurality of wavelengths and emitting the laser beams.

JP2007-235063A discloses only the structure which rotates the mechanical chopper to obtain the pulsed laser beam, but the switching of a plurality of wavelengths is not considered in JP2007-235063A. In JP2007-235063A, in order to obtain pulsed laser beams with a plurality of wavelengths, it is necessary to provide means for selecting a wavelength, independently from the mechanical chopper, which results in an increase in the number of components.

The invention has been made in view of the above-mentioned problems and an object of the invention is to provide a laser source unit that can obtain Q switch pulse oscillation with a simple structure while continuously switching a plurality of wavelengths. Another object of the invention is to provide a photoacoustic image generation apparatus including the laser source unit.

In order to achieve the objects of the invention, according to an aspect of the invention, there is provided a laser source unit that emits pulsed laser beams with a plurality of different wavelengths. The laser source unit includes: a laser rod; an excitation light source that radiates excitation light to the laser rod; an optical resonator including a pair of mirrors that face each other with the laser rod interposed therebetween; a wavelength selection means that controls a wavelength of light which resonates in the optical resonator to any one of the plurality of wavelengths; and driving means for driving the wavelength selection means such that the optical resonator performs Q switch pulse oscillation.

In the laser source unit according to the above-mentioned aspect of the invention, the wavelength selection means may be capable of rotary driving. With the rotary driving of the wavelength selection means, an insertion loss of the optical resonator may be changed from a first loss to a second loss which is less than the first loss.

The laser source unit according to the above-mentioned aspect of the invention may further include a light emission control unit that controls the excitation light source. The light emission control unit may direct the excitation light source to radiate the excitation light at a time that is a predetermined time before a time when the wavelength selection means switches the insertion loss of the optical resonator from the first loss to the second loss.

In the laser source unit according to the above-mentioned aspect of the invention, the excitation light source may be turned off at the same time as the wavelength selection means switches the insertion loss of the optical resonator from the first loss to the second loss.

In the laser source unit according to the above-mentioned aspect of the invention, when an upper limit of the number of times the optical resonator can perform the Q switch pulse oscillation while the wavelength selection means makes one rotation is m, a rotational frequency when the driving means rotary drives the wavelength selection means is F [rotations/second], and n is a predetermined natural number, the light emission control unit may direct the excitation light source to radiate the excitation light for mxF/n times per second.

In the laser source unit according to the above-mentioned aspect of the invention, a switching time when the insertion loss of the optical resonator is switched from the first loss to the second loss with the driving of the wavelength selection means may be less than a generation delay time of a Q switch pulse.

In the laser source unit according to the above-mentioned aspect of the invention, the wavelength selection means may include a filter rotating body that includes a plurality of transparent regions and non-transparent regions which are alternately arranged along a circumferential direction. The plurality of transparent regions may selectively transmit light components with predetermined wavelengths corresponding to the plurality of wavelengths. The driving means may continuously rotate the filter rotating body such that the non-transparent regions and the transparent regions are alternately inserted onto an optical path of the optical resonator. When the region inserted onto the optical path of the optical resonator is switched from the non-transparent region to the transparent region, the optical resonator may perform the Q switch pulse oscillation with a wavelength corresponding to the wavelength of the light which is transmitted by the switched transparent region. In this case, the filter rotating body may be rotated in a plane which is inclined at a predetermined angle with respect to an optical axis of the optical resonator. The transparent region may include a bandpass filter. The transparent region may have a fan shape or a circular shape.

Instead of the above-mentioned structure, the wavelength selection means may include a mirror rotating body that includes a plurality of reflection regions and regions that do not reflect light, which are alternately arranged along a circumferential direction. The plurality of reflection regions may selectively reflect light components with predetermined wavelengths corresponding to the plurality of wavelengths. The driving means may continuously rotate the mirror rotating body such that the regions that do not reflect light and the reflection regions are alternately inserted onto an optical path of the optical resonator. The reflection regions of the mirror rotating body may operate as one of the pair of mirrors. When the region which is inserted onto the optical path of the optical resonator is switched from the region that does not reflect light to the reflection region, the optical resonator may perform the Q switch pulse oscillation with a wavelength corresponding to the wavelength of the light which is reflected by the switched reflection region.

Alternatively, the wavelength selection means may include a mirror rotating body that includes a plurality of reflecting surfaces which function as one of the pair of mirrors. The plurality of reflecting surfaces selectively reflect light components with predetermined wavelengths corresponding to the plurality of wavelengths. The driving means may continuously rotate the mirror rotating body such that the reflecting surfaces which face the other of the pair of mirrors are sequentially switched. When the reflecting surface is perpendicular with respect to an optical axis of the optical resonator, the optical resonator may perform the Q switch pulse oscillation with a wavelength corresponding to the wavelength of the light which is reflected by the reflecting surface perpendicular with respect to the optical axis.

The laser source unit according to the above-mentioned aspect of the invention may further include a condensing lens that is provided within the optical resonator and reduces a beam diameter of a light which travels toward the wavelength selection means in the optical resonator.

The condensing lens may reduce the beam diameter of the light at the position of the wavelength selection means to 100 µm or less.

According to another aspect of the invention, a photoacoustic image generation apparatus includes: a laser source unit that emits pulsed laser beams with a plurality of different wavelengths and includes a laser rod, an excitation light source that radiates excitation light to the laser rod, an optical resonator including a pair of mirrors that face each other with the laser rod interposed therebetween, a wavelength selection means that controls a wavelength of light which resonates in the optical resonator to any one of the plurality of wavelengths, and a driving means that drives the wavelength selection means such that the optical resonator performs Q switch pulse oscillation; a detection means that detects a photoacoustic signal which is generated in a subject when the pulsed laser beams with the plurality of wavelengths are radiated to the subject and generating photoacoustic data corresponding to each wavelength; an intensity ratio extraction means that extracts a magnitude relationship between relative signal intensities of the photoacoustic data corresponding to each wavelength; and a photoacoustic image construction means that generates a photoacoustic image based on the extracted magnitude relationship.

In the photoacoustic image generation apparatus according to the above-mentioned aspect of the invention, the wavelength selection means may be capable of rotary driven to change an insertion loss of the optical resonator from a first loss to a second loss which is less than the first loss. The photoacoustic image generation apparatus may further include: a driving state detection means that detects a rotational position of the wavelength selection means; a rotation control unit that controls the driving means such that the wavelength selection means is rotated at a predetermined rotational speed; and a light emission control unit that directs the excitation light source to radiate the excitation light when the rotational position detected by the driving state detection means is a predetermined distance shorter than a rotational position where the wavelength selection means switches the insertion loss of the optical resonator from the first loss to the second loss.

The rotation control unit may control the driving means such that a variation in the rotational position detected by the driving state detection means for a predetermined time is constant.

The light emission control unit may generate a synchronous signal when the rotational position detected by the driving state detection means is in the rotational position where the wavelength selection means switches the insertion loss of the optical resonator from the first loss to the second loss, and the detection means may start to detect the photoacoustic signal based on the synchronous signal.

The photoacoustic image generation apparatus according to the above-mentioned aspect of the invention may further include an intensity information extraction means that generates intensity information indicating signal intensity based on the photoacoustic data corresponding to each wavelength. The photoacoustic image construction means may determine a gradation value of each pixel in the photoacoustic image based on the intensity information and determine a display color of each pixel based on the extracted magnitude relationship.

The plurality of wavelengths of the pulsed laser beams emitted by the laser source unit may include a first wavelength and a second wavelength. The photoacoustic image generation apparatus may further include: a complexification means that generates complex data in which one of first photoacoustic data which corresponds to the photoacoustic signal detected when the pulsed laser beam with the first wavelength is radiated and second photoacoustic data which corresponds to the photoacoustic signal detected when the pulsed laser beam with the second wavelength is radiated is a real part and the other photoacoustic data is an imaginary part; and a photoacoustic image reconstruction means that generates a reconstructed image from the complex data using a Fourier transform method. The intensity ratio extraction means may extract phase information as the magnitude relationship from the reconstructed image, and the intensity information extraction means may extract the intensity information from the reconstructed image.

The detection means may further detect a reflected acoustic wave when an acoustic wave is transmitted to the subject and generates reflected acoustic wave data. The photoacoustic image generation apparatus may further include an acoustic wave image generation means that generates an acoustic wave image based on the reflected acoustic wave data.

In the laser source unit according to the above-mentioned aspect of the invention, the wavelength selection means that controls the wavelength of light resonating in the optical resonator to any one of a plurality of wavelengths is driven such that the optical resonator performs Q switch pulse oscillation. In the above-mentioned aspect of the invention, since the wavelength selection means operates as a Q switch, it is not necessary to separately provide the Q switch and the wavelength selection means in the optical resonator. Therefore, it is possible to reduce the number of components.

### BRIEF DESCRIPTION OF THE DRAWINGS

Fig. 1 is a block diagram illustrating a photoacoustic image generation apparatus according to a first embodiment of the invention.
Fig. 2 is a block diagram illustrating the structure of a laser source unit according to the first embodiment.
Fig. 3 is a diagram illustrating an example of the structure of wavelength selection means.
Fig. 4 is a graph illustrating the relationship between a wavelength and transmittance in a transparent region.
Fig. 5 is a block diagram illustrating a portion of the laser source unit.
Figs. 6A to 6C are timing charts illustrating the light emission timing of a flash lamp and the timing of a pulsed laser beam.
Fig. 7 is a timing chart illustrating the emission of a pulsed laser beam.
Fig. 8 is a flowchart illustrating an operational procedure of the photoacoustic image generation apparatus according to the first embodiment.
Fig. 9 is a diagram illustrating another example of the structure of a filter rotating body.
Fig. 10 is a cross-sectional view illustrating a cross-section in the vicinity of the transparent region.
Fig. 11 is a diagram illustrating an example of the structure of the wavelength selection means when four wavelengths are switched and light is emitted.
Fig. 12 is a timing chart illustrating the emission of a pulsed laser beam.
Fig. 13 is a block diagram illustrating a portion of a laser source unit when the wavelength selection means also functions as a rear mirror.
Fig. 14 is a graph illustrating the relationship between a wavelength and reflectivity in a reflection region.
Fig. 15 is a block diagram illustrating a portion of a laser source unit which uses a mirror rotating body having two surfaces as the wavelength selection means.
Fig. 16 is a block diagram illustrating a portion of a laser source unit which uses a mirror rotating body (polyhedron) having five surfaces as the wavelength selection means.
Fig. 17 is a block diagram illustrating a photoacoustic image generation apparatus according to a second embodiment of the invention.
Fig. 18 is a block diagram illustrating an operational procedure of the photoacoustic image generation apparatus according to the second embodiment.
Figs. 19A to 19C are timing charts illustrating an example in which the frequency of Q switch repetition is lower than the rotational frequency of a filter rotating body.
Fig. 20 is a graph illustrating the molecular absorption coefficients of oxygenated hemoglobin and deoxygenated hemoglobin for each wavelength of light.

### DESCRIPTION OF THE PREFERRED EMBODIMENTS

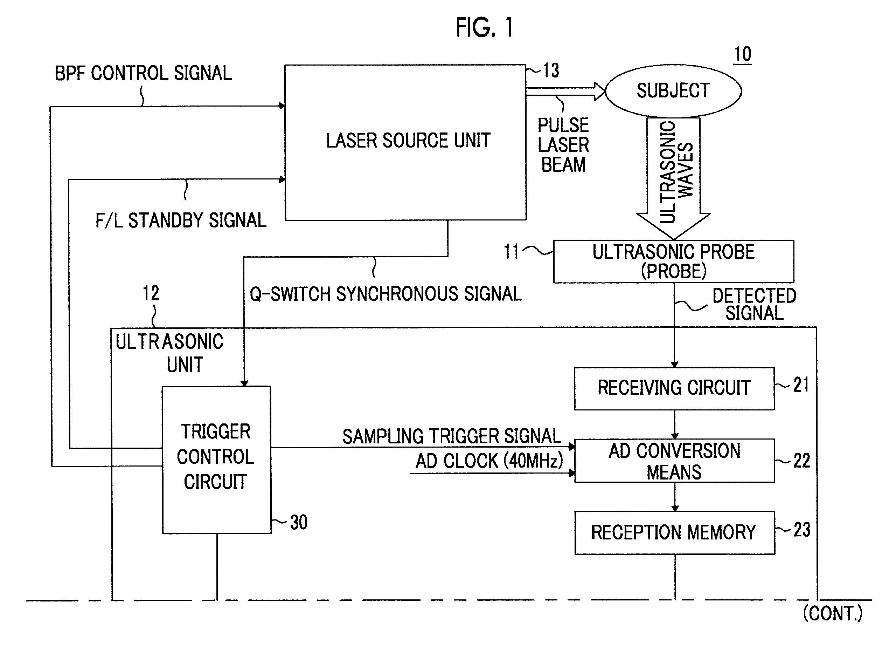

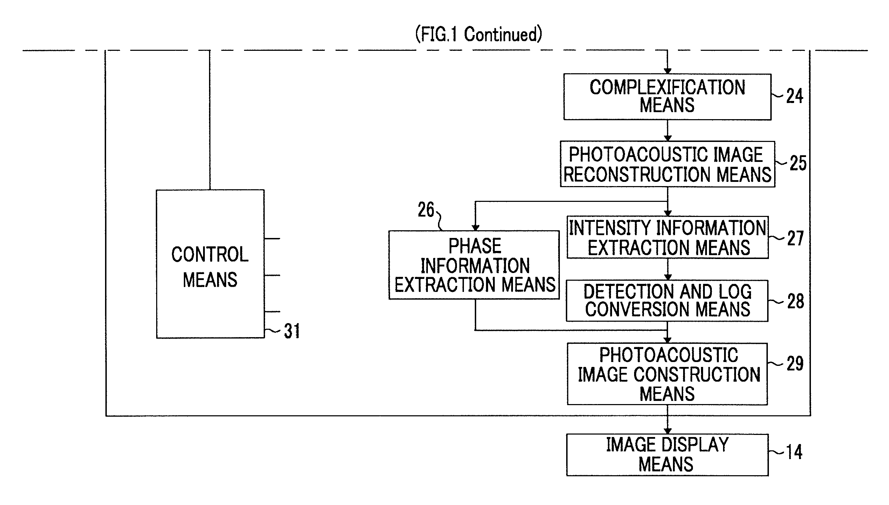

Hereinafter, embodiments of the invention will be described in detail with reference to the drawings. Fig. 1 shows a photoacoustic image generation apparatus according to a first embodiment of the invention. A photoacoustic image generation apparatus 10 includes an ultrasonic probe 11, an ultrasonic unit 12, and a laser source unit 13. The laser source unit 13 emits pulsed laser beams to be radiated to a subject. The laser source unit 13 switches the pulsed laser beams with a plurality of different wavelengths and emits the pulsed laser beams. In the following description, mainly, the laser source unit 13 sequentially emits a pulsed laser beam with a first wavelength and a pulsed laser beam with a second wavelength. In the embodiment of the invention, an ultrasonic wave is used as an acoustic wave. However, the acoustic wave is not limited to the ultrasonic wave, but an acoustic wave with an audio frequency may be used as long as an appropriate frequency can be selected depending on, for example, the subject or measurement conditions.

For example, it is considered that the first wavelength (central wavelength) is about 750 nm and the second wavelength is about 800 nm. Referring to the above-mentioned Fig. 20, a molecular absorption coefficient of oxygenated hemoglobin (hemoglobin combined with oxygen: oxy-Hb) which is contained in large quantities in human arteries, at a wavelength of 750 nm is less than a molecular absorption coefficient thereof at a wavelength of 800 nm. In contrast, a molecular absorption coefficient of deoxygenated hemoglobin (hemoglobin which is not combined with oxygen: deoxy-Hb) which is contained in large quantities in veins, at a wavelength of 750 nm is greater than a molecular absorption coefficient thereof at a wavelength of 800 nm. This property is used to check whether the level of a photoacoustic signal obtained at a wavelength of 750 nm is higher than that of a photoacoustic signal obtained at a wavelength of 800 nm. Therefore, it is possible to distinguish the photoacoustic signal obtained from the artery from the photoacoustic signal obtained from the vein.

The pulsed laser beam emitted from the laser source unit 13 is guided to the probe 11 by light guide means, such as an optical fiber, and is then radiated from the probe 11 to the subject. The radiation position of the pulsed laser beam is not particularly limited, but the pulsed laser beam may be radiated from a place other than the probe 11. In the subject, a light absorber absorbs the energy of the radiated pulsed laser beam and ultrasonic waves (acoustic waves) are generated. The probe 11 includes an ultrasonic detector. The probe 11 includes, for example, a plurality of ultrasonic detector elements (ultrasonic oscillators) which are one-dimensionally arranged and the ultrasonic oscillators which are one-dimensionally arranged detect the acoustic waves (photoacoustic signal) generated from the subject.

The ultrasonic unit 12 includes a receiving circuit 21, AD conversion means 22, a reception memory 23, a complexification means 24, photoacoustic image reconstruction means 25, phase information extraction means 26, intensity information extraction means 27, detection and log conversion means 28, photoacoustic image construction means 29, a trigger control circuit 30, and control means 31. The receiving circuit 21 receives the photoacoustic signal detected by the probe 11. The AD conversion means 22 is detection means, samples the photoacoustic signal received by the receiving circuit 21, and generates photoacoustic data which is digital data. The AD conversion means 22 samples the photoacoustic signal with a predetermined sampling period, in synchronization with an AD clock signal.

The AD conversion means 22 stores the photoacoustic data in the reception memory 23. The AD conversion means 22 stores photoacoustic data corresponding to each wavelength of the pulsed laser beams emitted from the laser source unit 13 in the reception memory 23. That is, the AD conversion means 22 stores, in the reception memory 23, first photoacoustic data obtained by sampling the photoacoustic signal detected by the probe 11 when the pulsed laser beam with the first wavelength is radiated to the subject and second photoacoustic data obtained by sampling the photoacoustic signal detected by the probe 11 when the pulsed laser beam with the second wavelength is radiated to the subject.

The complexification means 24 reads the first photoacoustic data and the second photoacoustic data from the reception memory 23 and generates complex data in which one of the first photoacoustic data and the second photoacoustic data is a real part and the other photoacoustic data is an imaginary part. In the following description, it is assumed that the complexification means 24 generates complex data in which the first photoacoustic data is a real part and the second photoacoustic data is an imaginary part.

The photoacoustic image reconstruction means 25 receives the complex data from the complexification means 24. The photoacoustic image reconstruction means 25 performs image reconstruction from the received complex data using a Fourier transform method (FTA method). For example, the method according to the related art disclosed in the document "Photoacoustic Image Reconstruction-A Quantitative Analysis" Jonathan I. Sperl et al. SPIE-OSA Vol. 6631 663103 can be used as the image reconstruction using the image Fourier transform method. The photoacoustic image reconstruction means 25 inputs Fourier transform data indicating a reconstructed image to the phase information extraction means 26 and the intensity information extraction means 27.

The phase information extraction means 26 extracts the magnitude relationship between the relative signal intensities of the photoacoustic data corresponding to each wavelength. In this embodiment, the phase information extraction means 26 receives input data as the reconstructed image obtained by the photoacoustic image reconstruction means 25 and extracts phase information indicating the magnitude relationship between the real part and the imaginary part from the input data which is complex data. For example, when the complex data is represented by X+iY, the phase information extraction means 26 generates θ=tan⁻¹(Y/X) as the phase information. It is assumed that, when X is 0, θ is 90°. When first photoacoustic data (X) forming the real part is equal to second photoacoustic data (Y) forming the imaginary part, the phase information is θ=45°. The phase information is close to θ=0° when the first photoacoustic data is relatively large and is close to θ=90° when the second photoacoustic data is relatively large.

The intensity information extraction means 27 generates intensity information indicating signal intensity based on photoacoustic data corresponding to each wavelength. In this embodiment, the intensity information extraction means 27 receives the reconstructed image obtained by the photoacoustic image reconstruction means 25 as input data and generates the intensity information from the input data which is complex data. For example, when the complex data is represented by X+iY, the intensity information extraction means 27 extracts (X²+Y²)^{1/2} as the intensity information. The detection and log conversion means 28 generates an envelope of data indicating the intensity information extracted by the intensity information extraction means 27, performs log conversion for the envelope, and expands a dynamic range.

The photoacoustic image construction means 29 receives the phase information from the phase information extraction means 26 and receives the intensity information subjected to the detection and log conversion process from the detection and log conversion means 28. The photoacoustic image construction means 29 generates a photoacoustic image, which is a light absorber distribution image, based on the received phase information and intensity information. The photoacoustic image construction means 29 determines the brightness (gradation value) of each pixel in the light absorber distribution image based on, for example, the received intensity information. In addition, the photoacoustic image construction means 29 determines the color (display color) of each pixel in the light absorber distribution image based on, for example, the phase information. The photoacoustic image construction means 29 determines the color of each pixel based on the received phase information, using, for example, a color map in which a phase range of 0° to 90° corresponds to a predetermined color.

In the phase range of 0° to 45°, the first photoacoustic data is more than the second photoacoustic data. Therefore, the source of the photoacoustic signal is considered to be the vein in which blood mainly including oxygenated hemoglobin that absorbs a larger amount of light energy at a wavelength of 756 nm than at a wavelength of 798 nm flows. In contrast, in the phase range of 45° to 90°, the second photoacoustic data is less than the first photoacoustic data. Therefore, the source of the photoacoustic signal is considered to be the artery in which blood mainly including deoxygenated hemoglobin absorbs a smaller amount of light energy at a wavelength of 756 nm than at a wavelength of 798 nm flows.

For example, the following color map is used: the color is blue at a phase of 0° and is gradually changed to an achromatic color (white) as the phase is close to 45°; and the color is red at a phase of 90° and is gradually changed to white as the phase is close to 45°. In this case, on the photoacoustic image, a portion corresponding to the artery can be represented in red and a portion corresponding to the vein can be represented in blue. The intensity information may not be used, the gradation value may be constant, and the portion corresponding to the artery and the portion corresponding to the vein may be represented in different colors based on the phase information. The image display means 14 displays the photoacoustic image generated by the photoacoustic image construction means 29 on a display screen.

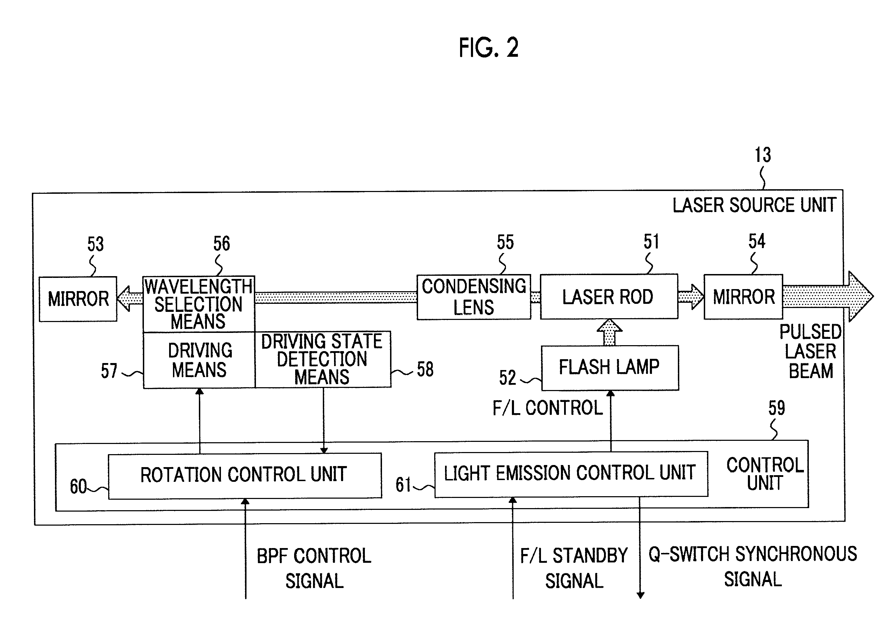

Then, the structure of the laser source unit 13 will be described in detail. Fig. 2 shows the structure of the laser source unit 13. The laser source unit 13 includes a laser rod 51, a flash lamp 52, mirrors 53 and 54, a condensing lens 55, wavelength selection means 56, driving means 57, driving state detection means 58, and a control unit 59. The laser rod 51 is a laser medium. For example, an alexandrite crystal, a Cr:LiSAF (Cr:LiSrAlF6) or Cr:LiCAF (Cr:LiCaAlF6) crystal, or a Ti:Sapphire crystal can be used as the laser rod 51. The flash lamp 52 is an excitation light source and radiates excitation light to the laser rod 51. A light source other than the flash lamp 52, for example, a semiconductor laser or a solid-state laser may be used as the excitation light source.

The mirrors 53 and 54 face each other with the laser rod 51 interposed therebetween. An optical resonator is formed by the mirrors 53 and 54. Here, it is assumed that the mirror 54 is an output-side mirror. The condensing lens 55 and the wavelength selection means 56 are arranged in the optical resonator. The wavelength selection means 56 controls the wavelength of light which resonates in the optical resonator to any one of a plurality of wavelengths to be emitted. The condensing lens 55 is arranged between the laser rod 51 and the wavelength selection means 56, condenses light which is incident from the laser rod 51, and emits the light to the wavelength selection means 56. That is, the condensing lens 55 reduces the beam diameter of the light which travels to the wavelength selection means 56 in the optical resonator.

The wavelength selection means 56 includes, for example, a plurality of transparent regions and non-transparent regions which are alternately arranged along the circumferential direction. The plurality of transparent regions selectively transmit light components with predetermined wavelengths corresponding to a plurality of wavelengths. The wavelength selection means 56 includes, for example, two transparent regions and two non-transparent regions. A first bandpass filter (BPF) which transmits light with, for example, a wavelength of 750 nm (central wavelength) is provided in one of the two transparent regions and a second bandpass filter which transmits light with a wavelength of 800 nm (central wavelength) is provided in the other transparent region.

The wavelength selection means 56 having the above-mentioned structure is rotated to selectively insert any one of the plurality of bandpass filters onto an optical path of the optical resonator. For example, the wavelength selection means 56 sequentially inserts the non-transparent region, the first bandpass filter, the non-transparent region, and the second bandpass filter onto the optical path of the optical resonator. The first bandpass filter is inserted onto the optical path of the optical resonator to set the oscillation wavelength of the light oscillator to 750 nm. The second bandpass filter is inserted onto the optical path of the optical resonator to set the oscillation wavelength of the light oscillator to 800 nm.

The wavelength selection means 56 is configured such that it is rotated to change the insertion loss of the optical resonator from a large loss (first loss) to a small loss (second loss). When the first or second bandpass filter is inserted onto the optical path of the optical resonator, the insertion loss of the optical resonator is small (high Q factor). When the non-transparent region is inserted onto the optical path of the optical resonator, the insertion loss of the optical resonator is large (low Q factor). The wavelength selection means 56 also functions as a Q switch. The wavelength selection means 56 is rotated to rapidly change the insertion loss of the optical resonator from a large loss (low Q factor) to a small loss (high Q factor). Therefore, it is possible to obtain a pulsed laser beam.

The driving means 57 drives the wavelength selection means 56 such that the optical resonator performs Q switch pulse oscillation. That is, the driving means 57 drives the wavelength selection means 56 so as to rapidly change the insertion loss of the optical resonator from a large loss (a low Q factor) to a small loss (a high Q factor). For example, when the wavelength selection means 56 includes a filter rotating body in which transparent regions (bandpass filters) and non-transparent regions are alternately arranged along the circumferential direction, the driving means 57 continuously rotates the filter rotating body such that the non-transparent regions and the transparent regions are alternately inserted onto the optical path of the optical resonator. It is preferable that the switching time when the insertion loss of the optical resonator is switched from a large loss to a small loss with the driving of the wavelength selection means 56 be less than the generation delay time of a Q switch pulse. When the region inserted onto the optical path of the optical resonator is switched from the non-transparent region to the transparent region (the first or second bandpass filter), it is possible to make the optical resonator perform Q switch pulse oscillation with a wavelength corresponding to the wavelength of light which is transmitted by the transparent region (bandpass filter) inserted onto the optical path.

The driving state detection means 58 detects the driving state of the wavelength selection means 56. The driving state detection means 58 detects, for example, the rotational displacement of the wavelength selection means 56 which includes the filter rotating body. The driving state detection means 58 outputs BPF state information indicating the rotational displacement of the filter rotating body to the control unit 59.

The control unit 59 includes a rotation control unit 60 and a light emission control unit 61. The rotation control unit 60 controls the driving means 58 such that the wavelength selection means 56 is rotated at a predetermined rotational speed. The rotational speed of the wavelength selection means 56 can be determined based on, for example, the number of wavelengths of the pulsed laser beams to be emitted from the laser source unit 13 (the number of bandpass filters in the filter rotating body) and the number of pulsed laser beams per unit time. The rotation control unit 59 controls the driving means 57 such that a variation in the rotational position detected by the driving state detection means 58 for a predetermined period of time is constant. For example, the rotation control unit 59 controls the driving means 57 such that a variation in the BPF state information for a predetermined period of time is a variation corresponding to the switching speed (the rotational speed of the filter rotating body) of a predetermined bandpass filter.

The light emission control unit 61 controls the flash lamp 52. The light emission control unit 61 outputs a flash lamp control signal to the flash lamp 52 such that the flash lamp 52 radiates excitation light to the laser rod 51. The light emission control unit 61 outputs the flash lamp control signal to the flash lamp 52 at the time that is a predetermined time before the time when the wavelength selection means 56 switches the insertion loss of the optical resonator from a large loss to a small loss such that the flash lamp 52 radiates the excitation light. That is, when the rotational position detected by the driving state detection means 58 is a predetermined distance shorter than the rotational position where the wavelength selection means 56 switches the insertion loss of the optical resonator from a large loss to a small loss, the light emission control unit 61 transmits the flash lamp control signal to the flash lamp 52 such that the flash lamp 52 radiates excitation light.

For example, when the BPF state information is information indicating a position obtained by subtracting the amount of displacement of the wavelength selection means 56 for the time required for the excitation of the laser rod 51 from the driving position of the wavelength selection means 56 where the bandpass filter corresponding to the wavelength of the pulsed laser beam to be emitted is inserted onto the optical path of the optical resonator, the light emission control unit 61 outputs the flash lamp control signal such that the flash lamp 52 radiates the excitation light to the laser rod 51. When the rotational position detected by the driving state detection means 58 is the rotational position where the wavelength selection means 56 switches the insertion loss of the optical resonator from a large loss to a small loss after the flash lamp control signal is output, the light emission control unit 61 generates a Q switch synchronous signal indicating the time when the Q switch is turned on and outputs the Q switch synchronous signal to the ultrasonic unit 12.

Returning to Fig. 1, the control means 31 controls each unit of the ultrasonic unit 12. The trigger control circuit 30 outputs a BPF control signal for controlling the rotational speed of the wavelength selection means 56 to the laser source unit 13. In addition, the trigger control circuit 30 outputs a flash lamp standby signal for controlling the emission of light from the flash lamp 52 to the laser source unit 13. For example, the trigger control circuit 30 receives the current rotational displacement position of the filter rotating body from the rotation control unit 60 of the laser source unit 13 and outputs the flash lamp standby signal at the time based on the received rotational displacement position.

The trigger control circuit 30 receives the Q switch synchronous signal indicating the time when the Q switch is turned on, that is, a laser beam emission time, from the laser source unit 13. When receiving the Q switch synchronous signal, the trigger control circuit 30 outputs a sampling trigger signal (AD trigger signal) to the AD conversion means 22. The AD conversion means 22 starts to sample the photoacoustic signal based on the sampling trigger signal.

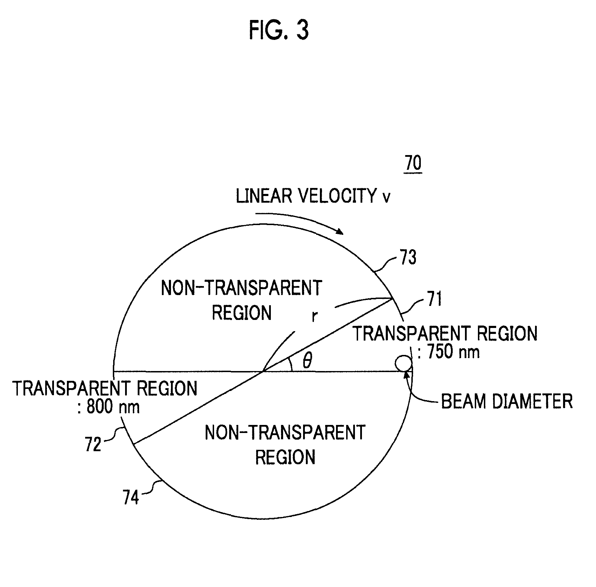

Fig. 3 shows an example of the structure of the wavelength selection means 56. The wavelength selection means 56 includes, for example, a filter rotating body 70 including a plurality of transparent regions (bandpass filters) with different transmission wavelengths shown in Fig. 3. The filter rotating body 70 includes a first transparent region 71 which selectively transmits light with a wavelength of 750 nm, a second transparent region 72 which selectively transmits a wavelength of 800 nm, and non-transparent regions 73 and 74 which do not transmit light. The non-transparent region does not necessarily require a capability to completely shield light. The non-transparent region may transmit a very small amount of light which does not cause unnecessary laser oscillation.

The first transparent region 71 and the second transparent region 72 each have, for example, a fan shape with a central angle θ. The light condensed by the condensing lens 55 (Fig. 2) is radiated to the circumference of the filter rotating body. When the filter rotating body 70 is rotated in the clockwise direction, the first transparent region 71, the non-transparent region 73, the second transparent region 72, and the non-transparent region 74 can be inserted in this order onto the optical path of the optical resonator. It is possible to obtain a pulsed laser beam with a wavelength which varies depending on a pulse by changing the wavelength of light which passes through each of the first transparent region 71 and the second transparent region 72, that is, by changing the transmission wavelength of the bandpass filter provided in each transparent region.

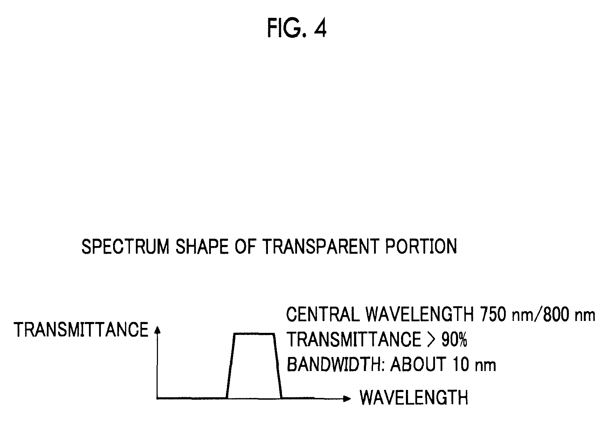

Fig. 4 shows the relationship between the wavelength and transmittance in the transparent region. It is assumed that the transmittance of the first transparent region (first bandpass filter) 71 with respect to light with a central wavelength of 750 nm is equal to or greater than 90%. The band width is about 10 nm. The transmittance of the second transparent region (second bandpass filter) 72 with respect to light with a central wavelength of 800 nm is equal to or greater than 90%. The band width is about 10 nm.

Here, it is assumed that the rotational frequency of the filter rotating body 70 is 100 Hz (rotational speed: 6000 rpm). In this case, since light passes through two transparent regions during one rotation, the laser source unit 13 emits 200 pulsed laser beams per second (200-Hz operation). A filter rotating body with a radius of 2 inches (50.4 mm) is considered as the filter rotating body 70. In addition, it is assumed that a beam diameter of the light is 100 µm. An angular velocity ω is 2πf = 628.3 [rad/sec] and a linear velocity v is rω = 628.8 [rad/sec]×50.4 [mm] = 31.7 [m/s]. The time required to cross the beam (switching time) is 3.15 µsec.

As the characteristics of the Q switch, the condition for obtaining a single pulse is that the switching time (for example, the switching time from the non-transparent region to the first or second transparent region) is about a few microseconds or less (which is shorter than the generation delay time of the Q switch pulse). The central angle θ of the transparent region is selected on condition that the beam is not hindered for the sum of the time required to cross the beam and the Q switch delay time. In the above-mentioned numerical example, the transparent region may continue for 10 µsec = 3.15 µsec + a few microseconds. The width of the region is 317 µm = 31.7 [m/s]x 10 µsec and corresponds to an angle of 0.35°. The central angle θ may be in the range of 1° to a few degrees in view of manufacturing.

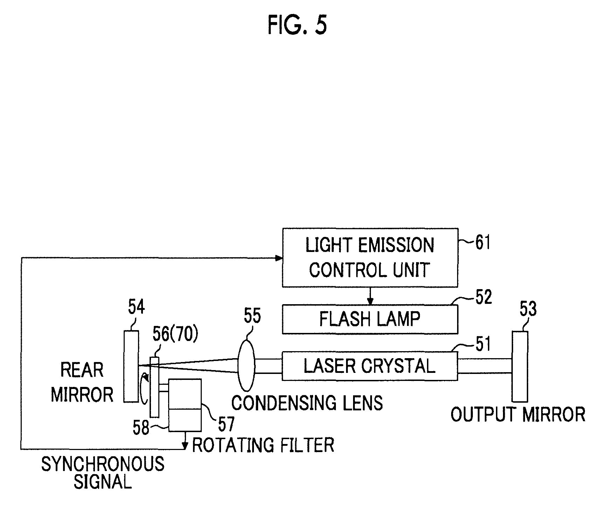

Fig. 5 shows a portion of the laser source unit 13. The wavelength selection means 56 is, for example, the filter rotating body 70 including two bandpass filters (two transparent regions which transmit light components with different wavelengths) shown in Fig. 3. It is preferable that the beam diameter of the light on the filter rotating body 70 be small. In this embodiment, the condensing lens 55 is used to condense a beam. It is preferable that the beam diameter of the light on the filter rotating body 70 be equal to or less than 100 µm. The lower limit of the beam diameter of the light is determined by a diffraction limit and is a few micrometers (µmφ). The filter rotating body 70 is inclined at an angle of, for example, about 0.5° to 1° with respect to the optical axis of the optical resonator so as to be rotated in a plane which is inclined at a predetermined angle with respect to the optical axis. As such, when the filter rotating body 70 is slightly inclined with respect to the optical axis of the optical resonator, it is possible to prevent the parasitic oscillation of an unnecessary reflection component.

The driving means 57 is, for example, a servomotor and rotates the wavelength selection means 56 (filter rotating body 70) about a rotating shaft. It is preferable that the rotational frequency of the filter rotating body 70 be high. Mechanically, the rotational frequency of the filter rotating body 70 can be up to about 1 kHz. The driving state detection means 58 is, for example, a rotary encoder. The rotary encoder detects the rotational displacement of the filter rotating body using a rotating plate with a slit which is attached to an output shaft of the servomotor and a transmissive photointerrupter and converts the rotation of the filter rotating body 70 into an electric signal (BPF state signal). The rotary encoder uses the electric signal as a master clock and transmits the electric signal as a synchronous signal to the light emission control unit 61. The light emission control unit 61 determines the light emission timing of the flash lamp based on the rotation of the filter rotating body 70 which is being rotated with high accuracy.

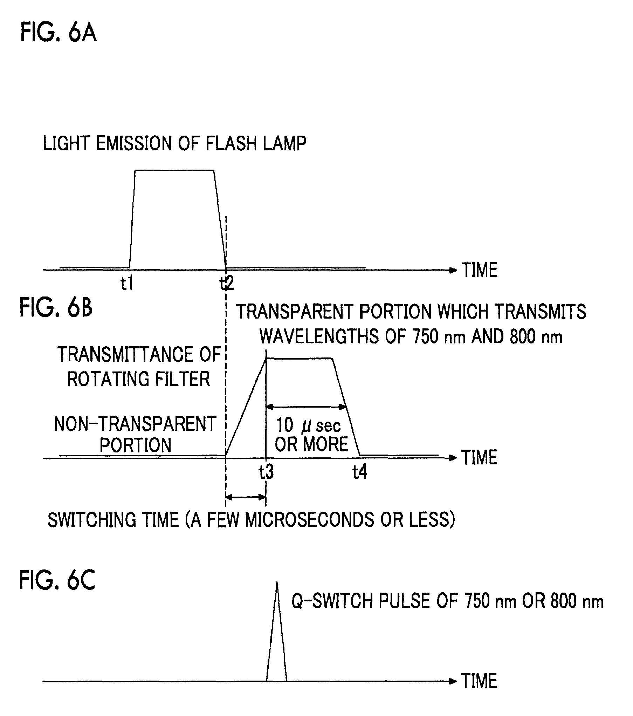

Figs. 6A to 6C show the light emission timing of the flash lamp and the timing of the pulsed laser beam. A time t2 corresponds to a rotational position where the filter rotating body 70 (wavelength selection means 56) which is being rotated is switched from the non-transparent region to the transparent region. A time t1 is obtained by subtracting the time required to excite the laser rod 51 from the time t2. When the rotational position of the filter rotating body 70 is a position corresponding to the time t1, the light emission control unit 61 directs the flash lamp 52 to emit light (Fig. 6A). When the flash lamp 52 emits light, the laser rod 51 is excited.

After the flash lamp emits light, at the time t2, the filter rotating body 70 is switched from the non-transparent region to the transparent region (the first transparent region 71 or the second transparent region 72) at the same time as the flash lamp is turned off (Fig. 6B). Here, the term "same time" includes substantially the same time and means that there is no influence on the generation of the pulsed laser beam for the time from the turn-off of the flash lamp to the switching of the filter rotating body 70 from the non-transparent region to the transparent region. The time (switching time) required for the switching from the non-transparent region to the transparent region may be as short as possible. The switching time is equal to or less than a few microseconds and preferably, equal to or less than 0.5 µsec. When the transparent region which transmits light with a wavelength of 750 nm is inserted onto the optical path of the optical resonator, the Q switch pulse oscillation of the light with a wavelength of 750 nm occurs at a time t3 and a pulsed laser beam with a wavelength of 750 nm is obtained (Fig. 6C). In contrast, when the transparent region which transmits light with a wavelength of 800 nm is inserted onto the optical path of the optical resonator, the Q switch pulse oscillation of the light with a wavelength of 800 nm occurs and a pulsed laser beam with a wavelength of 800 nm is obtained. The filter rotating body 70 is kept in the transparent region for about 10 µsec and is switched to the non-transparent region again at a time t4.

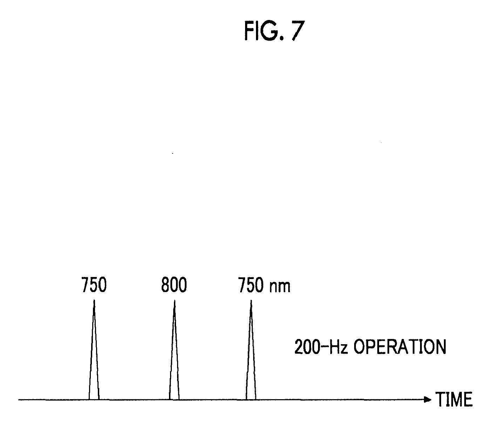

Fig. 7 shows the radiation of the pulsed laser beam. When the filter rotating body 70 shown in Fig. 3 in which the first transparent region 71 and the second transparent region 72 are provided between the non-transparent regions 73 and 74 is used, it is possible to obtain the pulsed laser beam with a wavelength which is switched between a wavelength of 750 nm and a wavelength of 800 nm for each pulse, as shown in Fig. 7. When the rotational frequency of the filter rotating body is 100 Hz, it is possible to obtain 200 pulsed laser beams for one second while switching the wavelength.

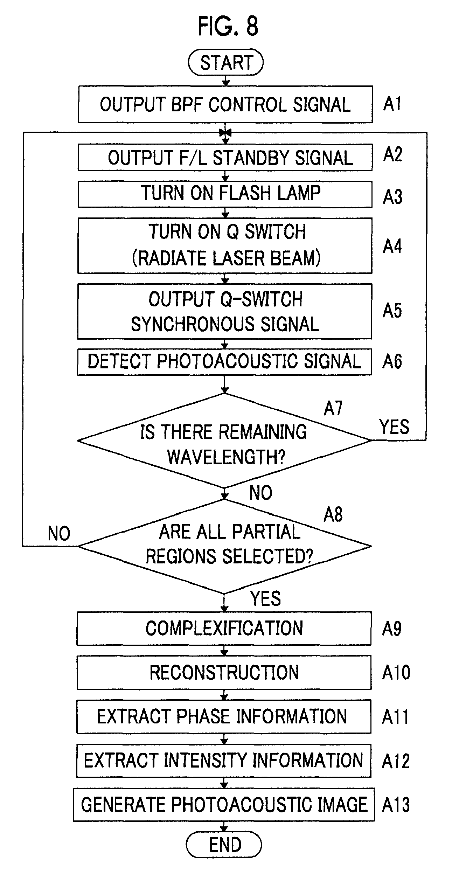

Fig. 8 shows the operational procedure of the photoacoustic image generation apparatus 10. In the following description, it is assumed that a region of the subject which is irradiated with a laser beam is divided into a plurality of partial regions. The trigger control circuit 30 outputs a BPF control signal for rotating the wavelength selection means (filter rotating body) 56 of the laser source unit 13 at a predetermined rotational speed to the laser source unit 13, prior to the radiation of the pulsed laser beam to the subject (Step A1).

When the trigger control circuit 30 is ready to receive a photoacoustic signal, it outputs a flash lamp standby signal to the laser source unit 13 at a predetermined time in order to radiate a pulsed laser beam with the first wavelength (for example, 750 nm) (Step A2). After receiving the flash lamp standby signal, the light emission control unit 61 of the laser source unit 13 transmits a flash lamp control signal to the flash lamp 52 to turn on the flash lamp 52 (Step A3). The light emission control unit 61 outputs the flash lamp control signal based on BPF state information, for example, at the time that is calculated back from the time when the rotational displacement position of the wavelength selection means 56 is switched from the non-transparent region 74 (Fig. 3) to the first transparent region 71 which transmits light with a wavelength of 750 nm. When the flash lamp 52 is turned on, the excitation of the laser rod 51 starts.

After the flash lamp is turned on, the wavelength selection means 56 is continuously rotated. When the portion which is inserted onto the optical path of the optical resonator is switched from the non-transparent region 74 to the first transparent region 71, the insertion loss of the optical resonator is rapidly changed from a large loss (low Q factor) to a small loss (high Q factor) and Q switch pulse oscillation occurs (Step A4). In this case, since the first transparent region 71 selectively transmits light with a wavelength of 750 nm, the laser source unit 13 emits a pulsed laser beam with a wavelength of 750 nm. The light emission control unit 61 outputs, to the ultrasonic unit 12, a Q switch synchronous signal indicating the time when the Q switch is turned on, that is, the time when the pulsed laser beam is radiated (Step A5).

The pulsed laser beam with a wavelength of 750 nm which is emitted from the laser source unit 13 is guided to, for example, the probe 11 and is radiated from the probe 11 to the first partial region of the subject. In the subject, a light absorber absorbs the energy of the radiated pulsed laser beam and a photoacoustic signal is generated. The probe 11 detects the photoacoustic signal generated from the subject. The photoacoustic signal detected by the probe 11 is received by the receiving circuit 21.

When receiving the Q switch synchronous signal, the trigger control circuit 30 outputs a sampling trigger signal to the AD conversion means 22. The AD conversion means 22 samples the photoacoustic signal received by the receiving circuit 21 with a predetermined sampling period (Step A6). The photoacoustic signal sampled by the AD conversion means 22 is stored as first photoacoustic data in the reception memory 23.

The control means 31 determines whether there is a remaining wavelength, that is, whether all of the pulsed laser beams with a plurality of wavelengths to be emitted are emitted (Step A7). When there is a remaining wavelength, the process returns to Step A2 in order to emit a pulsed laser beam with the next wavelength and the trigger control circuit 30 outputs the flash lamp standby signal to the laser source unit 13. The light emission control unit 61 transmits the flash lamp control signal to the flash lamp 52 to turn on the flash lamp 52 in Step A3. After the flash lamp is turned on, the portion which is inserted onto the optical path of the optical resonator is switched from the non-transparent region 73 to the second transparent region 72 corresponding to the second wavelength (800 nm) in Step A4 and Q switch pulse oscillation occurs. Then, the pulsed laser beam with a wavelength of 800 nm is radiated. The light emission control unit 61 outputs the Q switch synchronous signal to the ultrasonic unit 12 in Step A5.

The pulsed laser beam with a wavelength of 800 nm which is emitted from the laser source unit 13 is guided to, for example, the probe 11 and is radiated from the probe 11 to the first partial region of the subject. A light absorber in the subject absorbs the pulsed laser beam with a wavelength of 800 nm and a photoacoustic signal is generated. The probe 11 detects the generated photoacoustic signal. When receiving the Q switch synchronous signal, the trigger control circuit 30 outputs the sampling trigger signal to the AD conversion means 22. The AD conversion means 22 samples the photoacoustic signal in Step A6. The photoacoustic signal sampled by the AD conversion means 22 is stored as second photoacoustic data in the reception memory 23. The photoacoustic image generation apparatus 10 performs Steps A1 to A6 for each of the wavelengths of the pulsed laser beams to be radiated to the subject, radiates the pulsed laser beams with each wavelength to the subject, and detects the photoacoustic signal from the subject.

When it is determined in Step A7 that there is no remaining wavelength, the control means 31 determines whether all of the partial regions are selected (Step A8). When the partial region to be selected remains, the process returns to Step A2. The photoacoustic image generation apparatus 10 performs Steps A2 to A7 for each partial region, sequentially radiates the pulsed laser beams with each wavelength (750 nm and 800 nm) to each partial region, and stores the first photoacoustic data and the second photoacoustic data corresponding to each partial region in the reception memory 23. When the radiation of the pulsed laser beams and the detection of the photoacoustic signal are performed for all of the partial regions, photoacoustic data required to generate one frame of a photoacoustic image is obtained.

When it is determined in Step A8 that all of the partial regions are selected, the control means 31 proceeds to a photoacoustic image generation process. The complexification means 24 reads the first photoacoustic data and the second photoacoustic data from the reception memory 23 and generates complex data in which the first photoacoustic image data is a real part and the second photoacoustic image data is an imaginary part (Step A9). The photoacoustic image reconstruction means 25 reconstructs an image from the complex data which is complexified in Step A8 using the Fourier transform method (FTA method) (Step A10).

The phase information extraction means 26 extracts the phase information from the reconstructed complex data (reconstructed image) (Step A11). For example, when the reconstructed complex data is represented by X+iY, the phase information extraction means 26 extracts θ=tan⁻¹(Y/X) as the phase information (when X is 0, θ is 90°). The intensity information extraction means 27 extracts the intensity information from the reconstructed complex data (Step A12). For example, when the reconstructed complex data is represented X+iY, the intensity information extraction means 27 extracts (X²+Y²)^{1/2} as the intensity information.

The detection and log conversion means 28 performs a detection and log conversion process for the intensity information extracted in Step A12. The photoacoustic image construction means 29 generates a photoacoustic image based on the phase information extracted in Step A11 and the result of the detection and log conversion process for the intensity information extracted in Step A12 (Step A13). The photoacoustic image construction means 29 determines the brightness (gradation value) of each pixel in a light absorber distribution image based on, for example, the intensity information, determines the color of each pixel based on the phase information, and generates a photoacoustic image. The generated photoacoustic image is displayed on the image display means 14.

In this embodiment, the wavelength selection means 56 which controls the wavelength of light resonating in the optical resonator to any one of a plurality of wavelengths is driven such that the optical resonator performs Q switch pulse oscillation. For example, the wavelength selection means 56 including two bandpass filters with different transmission wavelengths are continuously driven to continuously and selectively insert the two bandpass filters onto the optical path of the optical resonator. It is possible to continuously switch the laser beams with a plurality of wavelengths and to emit the laser beams from the laser source unit 13. In this embodiment, since the wavelength selection means 56 also operates as a Q switch, it is possible to obtain Q switch pulse oscillation, without providing a separate Q switch in the optical resonator. In this embodiment, since the Q switch and the wavelength selection means do not need to be separately provided in the optical resonator, it is possible to reduce the number of components.

In this embodiment, the complex data in which one of the first photoacoustic data and the second photoacoustic data obtained from two wavelengths is a real part and the other photoacoustic data is an imaginary part is generated and the reconstructed image is generated from the complex data by the Fourier transform method. In this case, it is possible to effectively perform image reconstruction, as compared to a case in which the first photoacoustic data and the second photoacoustic data are individually reconstructed. The pulsed laser beams with a plurality of wavelengths are radiated and the photoacoustic signal (photoacoustic data) obtained when the pulsed laser beam with each wavelength is used. Therefore, it is possible to perform functional imaging using the fact that the light absorption characteristics of each light absorber vary depending on a wavelength.

In this embodiment, for example, when a light radiation region is divided into three partial regions, a pulsed laser beam with the first wavelength and a pulsed laser beam with the second wavelength are sequentially radiated to a first partial region. Then, the pulsed laser beam with the first wavelength and the pulsed laser beam with the second wavelength are sequentially radiated to a second partial region. Then, the pulsed laser beam with the first wavelength and the pulsed laser beam with the second wavelength are sequentially radiated to a third partial region. In this embodiment, the pulsed laser beam with the first wavelength and the pulsed laser beam with the second wavelength are consecutively radiated to a given partial region and are then radiated to the next partial region. In this case, it is possible to reduce the time from the radiation of the pulsed laser beam with the first wavelength to the radiation of the pulsed laser beam with the second wavelength at the same position, as compared to a case in which the pulsed laser beam with the first wavelength is radiated to three partial regions and then the pulsed laser beam with the second wavelength is radiated to the three partial regions. Since the time from the radiation of the pulsed laser beam with the first wavelength to the radiation of the pulsed laser beam with the second wavelength is reduced, it is possible to prevent the inconsistency of the first photoacoustic data and the second photoacoustic data.

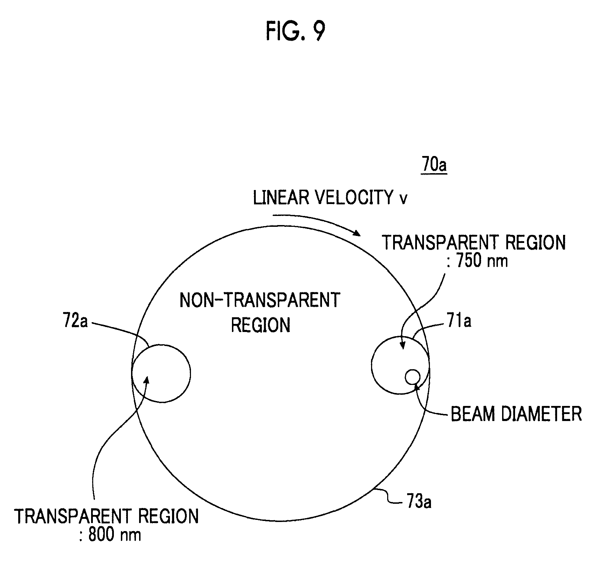

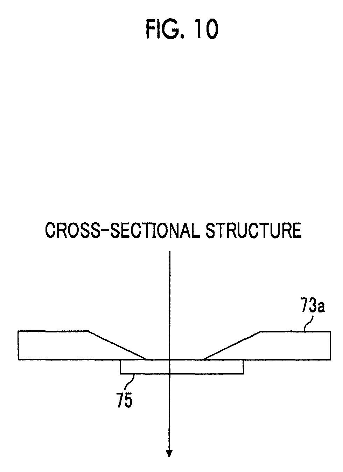

Fig. 9 shows another example of the structure of the filter rotating body. In Fig. 3, the transparent region has a fan shape. However, the shape of the transparent region is not limited to the fan shape. For example, as shown in Fig. 9, the first transparent region 71a and the second transparent region 72a may have a circular shape. The filter rotating body 70a is, for example, a metal plate forming the non-transparent region 73a. Black finishing may be performed for the front surface (particularly, the laser rod side) of the metal plate to reduce the reflectivity of the metal plate. Fig. 10 shows the cross-section of the vicinity of the transparent region. A plurality of openings may be provided in the metal plate forming the non-transparent region 73a and light filters 75 corresponding to each wavelength may be attached to each opening to form the first transparent region 71a and the second transparent region 72a. The size of the opening (transparent region) may be at least three to five times more than the beam diameter of the light. The size of the opening may be more than five times the beam diameter of the light.

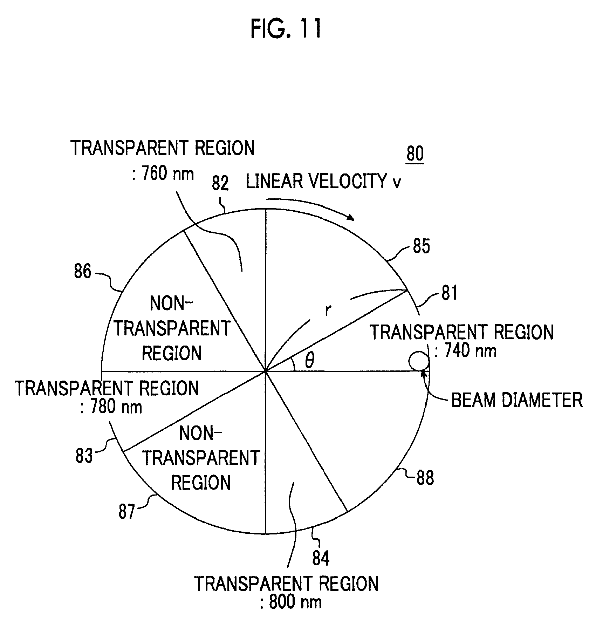

The number of transparent regions in the wavelength selection means (filter rotating body) 56, that is, the number of wavelengths of the laser beams emitted by the laser source unit 13 is not limited to two. The laser source unit 13 may switch pulsed laser beams with three or more wavelengths for each pulse and emit the pulsed laser beams. Fig. 11 shows an example of the structure of the wavelength selection means 56 when pulsed laser beams with four wavelengths are switched and emitted. In this example, the wavelength selection means 56 includes a filter rotating body 80 that includes four transparent regions 81 to 84 with different transmission wavelengths and four non-transparent regions 85 to 88. For example, the first transparent region 81 selectively transmits light with a wavelength of 740 nm and the second transparent region 82 selectively transmits light with a wavelength of 760 nm. The third transparent region 83 selectively transmits light with a wavelength of 780 nm and the fourth transparent region 84 selectively transmits light with a wavelength of 800 nm. In the filter rotating body 80, the transparent regions and the non-transparent regions are alternately arranged.

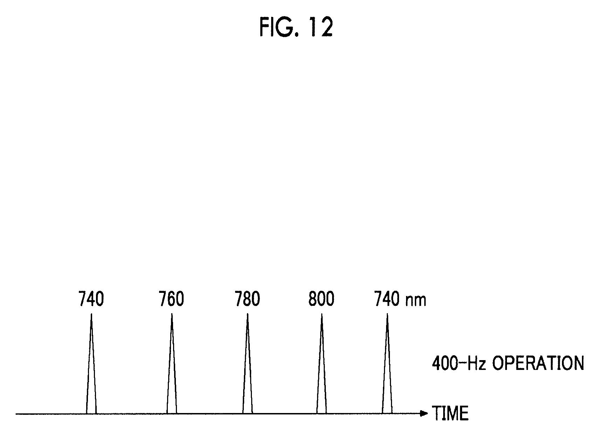

The filter rotating body 80 is rotated in the clockwise direction to sequentially insert the first transparent region 81, the non-transparent region 85, the second transparent region 82, the non-transparent region 86, the third transparent region 83, the non-transparent region 87, the fourth transparent region 84, and the non-transparent region 88 in this order onto the optical path of the optical resonator. Fig. 12 shows the emission of the pulsed laser beams. When the filter rotating body 80 shown in Fig. 11 in which the four transparent regions 81 to 84 are provided between the four non-transparent regions 85 to 88 is used, it is possible to obtain a pulsed laser beam with a wavelength that is sequentially changed to 740 nm, 760 nm, 780 nm, and 800 nm for each pulse, as shown in Fig. 12. When the rotational frequency of the filter rotating body is 100 Hz, it is possible to obtain 400 pulsed laser beams for one second while switching the wavelengths (400-Hz operation).

The number of transparent regions in the wavelength selection means (filter rotating body) 56 is not necessarily equal to the number of wavelengths of the pulsed laser beams emitted by the laser source unit 13. For example, in the filter rotating body 80 having four transparent regions shown in Fig. 11, the first transparent region 81 and the third transparent region 83 may selectively transmit light with the same wavelength and the second transparent region 82 and the fourth transparent region 84 may selectively transmit light with the same wavelength. Specifically, the first transparent region 81 and the third transparent region 83 may selectively transmit light with a wavelength of 750 nm and the second transparent region 82 and the fourth transparent region 84 may selectively transmit light with a wavelength of 800 nm. In this case, while the filter rotating body makes one rotation, it is possible to obtain four pulsed laser beams with wavelengths that are switched between 750 nm and 800 nm for each pulse.

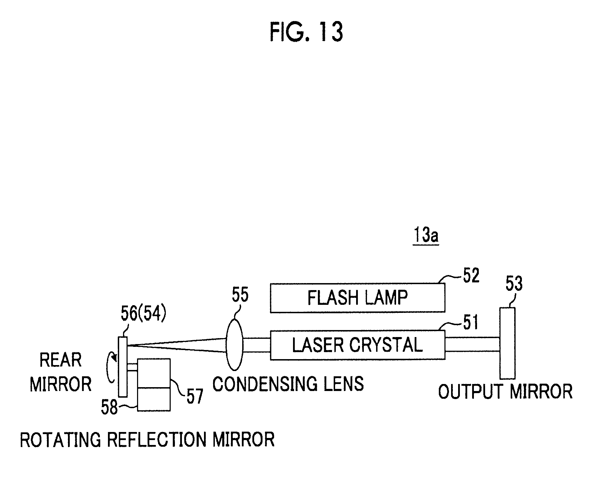

In the above-described embodiment, as the wavelength selection means 56, the filter is arranged between a pair of mirrors 53 and 54 forming the optical resonator. However, the invention is not limited thereto. The wavelength selection means 56 may also operate as one (for example, the rear mirror) of the pair of mirrors forming the optical resonator. Fig. 13 shows a portion of a laser source unit 13a when the wavelength selection means 56 also operate as the rear mirror. The wavelength selection means 56 is configured as, for example, a mirror rotating body including a plurality of reflection regions and regions that do not reflect light, which are alternately arranged along the circumferential direction. The plurality of reflection regions of the mirror rotating body selectively reflect light components with predetermined wavelengths corresponding to a plurality of wavelengths. The reflection regions operate as the rear mirror 54 of the optical resonator.

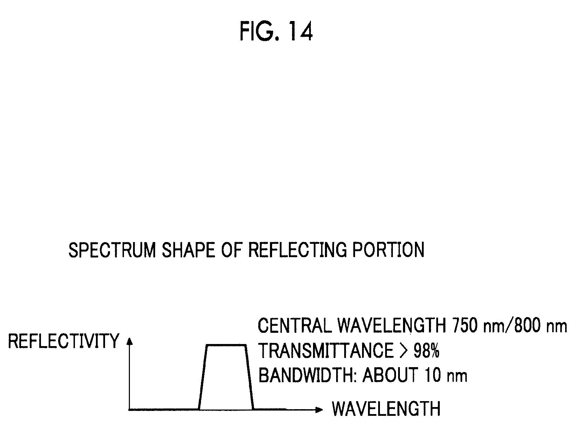

The mirror rotating body can be configured by replacing the transparent regions of the filter rotating body shown in Fig. 3 with the reflection regions and replacing the non-transparent regions with the regions that do not reflect light. Fig. 14 shows the relationship between the wavelength and reflectivity of the reflection region. For example, when pulsed laser beams with a wavelength of 750 nm and a wavelength of 800 nm are switched and emitted, the first reflection region may selectively reflect light with a wavelength of 750 nm and the second reflection region may selectively reflect light with a wavelength of 800. It is assumed that the reflectivity of each reflection region with respect to light with a wavelength of 750 nm and light with a wavelength of 800 nm is equal to or greater than 98%. The bandwidth of each reflection region is about 10 nm.

The driving means 57 continuously rotates the wavelength selection means (mirror rotating body) in the plane perpendicular with respect to the optical axis of the optical resonator. The driving means 57 rotates the mirror rotating body such that the regions which do not reflect light and the reflection regions in the mirror rotating body are alternately inserted onto the optical path of the optical resonator. The reflection region may have a fan shape which is the same as that of the transparent region shown in Fig. 3 or it may have a circular shape which is the same as that of the transparent region shown in Fig. 9. When the region which is inserted onto the optical path of the optical resonator is switched from the region which does not reflect light to the reflection region with the driving of the mirror rotating body, the insertion loss of the optical resonator is rapidly changed from a large loss to a small loss and Q switch pulse oscillation occurs. In this case, the optical resonator performs Q switch pulse oscillation with a wavelength corresponding to the wavelength of light reflected from the switched reflection region.

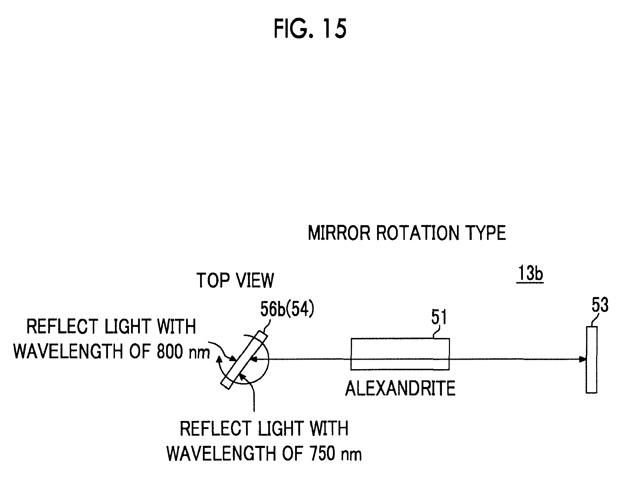

In the above-mentioned example, the mirror rotating body in which the reflection regions and the regions that do not reflect light are alternately arranged is used. However, the following mirror rotating body may be used: the mirror rotating body includes a plurality of reflecting surfaces that will function as the rear mirror; and the plurality of reflecting surfaces selectively reflect light components with predetermined wavelengths corresponding to a plurality of wavelengths. Fig. 15 shows a portion of a laser source unit 13b which uses a mirror rotating body having two surfaces as a wavelength selection means. One surface of wavelength selection means (mirror rotating body) 56b selectively reflects light with a wavelength of 750 nm and the other surface thereof selectively reflects light with a wavelength of 800 nm.

The driving means 57 continuously rotates the mirror rotating body such that the reflecting surfaces which face the mirror (output mirror) 53 are sequentially switched. For example, when the mirror rotating body is rotated and the reflecting surface which reflects light with a wavelength of 750 nm is perpendicular with respect to the optical axis of the optical resonator, light with a wavelength of 750 nm resonates in the optical resonator. When the reflecting surface which reflects light with a wavelength of 800 nm is perpendicular with respect to the optical axis of the optical resonator, light with a wavelength of 800 nm resonates in the optical resonator. Resonance does not occur at the other angles. In this driving method, it is also possible to rapidly switch the insertion loss of the optical resonator from a large loss to a small loss and to make the optical resonator perform Q switch pulse oscillation with a wavelength corresponding to the wavelength of light reflected from the reflecting surface which is perpendicular with respect to the optical axis.

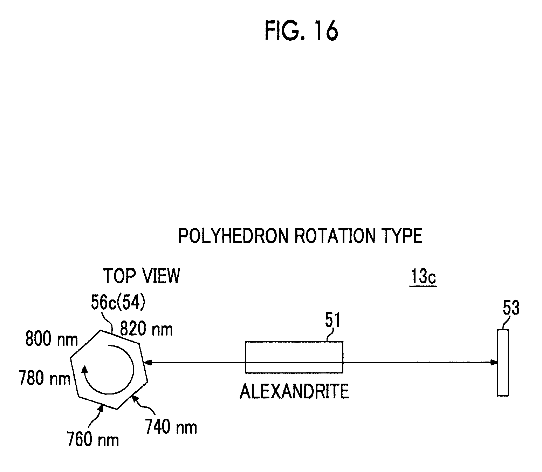

Fig. 16 shows a portion of a laser source unit 13c which uses a mirror rotating body (polyhedron) having five surfaces as a wavelength selection means. A wavelength selection means (mirror rotating body) 56c includes a surface which selectively reflects light with a wavelength of 740 nm, a surface which selectively reflects light with a wavelength of 760 nm, a surface which selectively reflects light with a wavelength of 780 nm, a surface which selectively reflects light with a wavelength of 800 nm, and a surface which selectively reflects light with a wavelength of 820 nm. The driving means 57 continuously rotates the wavelength selection means (mirror rotate polyhedron) 56c such that the reflecting surfaces facing the mirror 53 are sequentially switched. In this way, it is possible to obtain a pulsed laser beam with a wavelength that is sequentially changed to 820 nm, 800 nm, 780 nm, 760 nm, 740 nm, and 720 nm for each pulse.

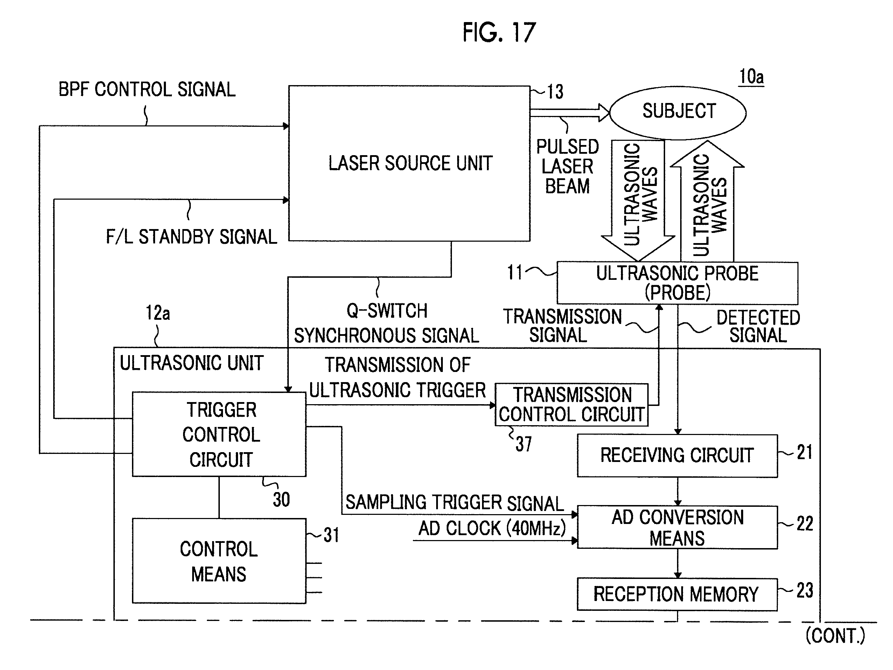

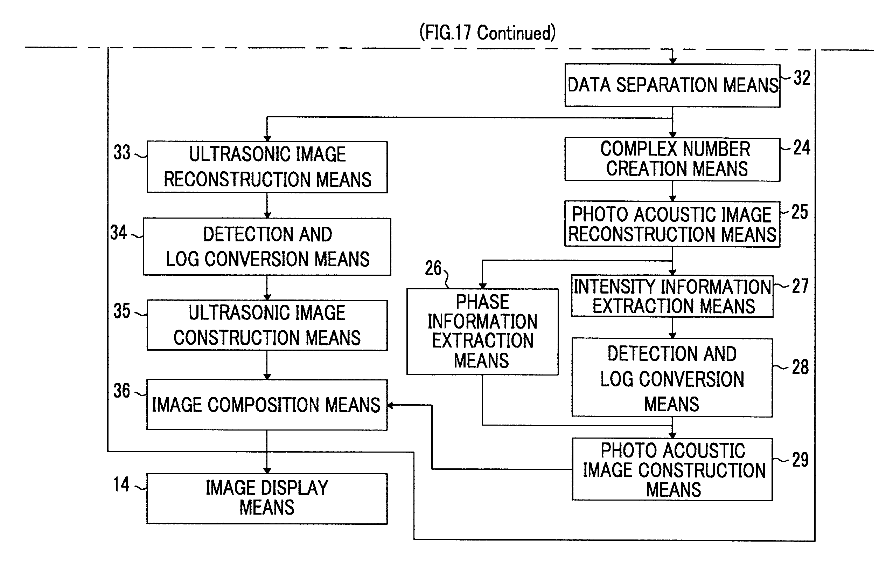

Next, a second embodiment of the invention will be described. Fig. 17 shows a photoacoustic image generation apparatus according to a second embodiment of the invention. In a photoacoustic image generation apparatus 10a according to this embodiment, an ultrasonic unit 12a includes data separation means 32, ultrasonic image reconstruction means 33, detection and log conversion means 34, ultrasonic image construction means 35, image composition means 36, and a transmission control circuit 37, in addition to the structure of the ultrasonic unit 12 in the photoacoustic image generation apparatus 10 according to the first embodiment shown in Fig. 1. The photoacoustic image generation apparatus 10a according to this embodiment differs from that according to the first embodiment in that it generates an ultrasonic image, in addition to the photoacoustic image. The other structures may be the same as those in the first embodiment.

In this embodiment, a probe 11 outputs (transmits) ultrasonic waves to the subject and detects (receives) ultrasonic waves reflected from the subject when the ultrasonic waves are transmitted to the subject, in addition to detecting the photoacoustic signal. A trigger control circuit 30 transmits an ultrasonic transmission trigger signal which instructs a transmission control circuit 37 to transmit ultrasonic waves when the ultrasonic image is generated. When receiving the trigger signal, the transmission control circuit 37 directs the probe 11 to transmit ultrasonic waves. The probe 11 detects ultrasonic waves reflected from the subject after transmitting ultrasonic waves.

The reflected ultrasonic waves detected by the probe 11 are input to AD conversion means 22 through a receiving circuit 21. The trigger control circuit 30 transmits a sampling trigger signal to the AD conversion means 22 in synchronization with the ultrasonic transmission time to start the sampling of the reflected ultrasonic waves. The AD conversion means 22 stores the sampling data (reflected ultrasonic data) of the reflected ultrasonic waves in a reception memory 23.

The data separation means 32 separates the reflected ultrasonic data stored in the reception memory 23 from first and second photoacoustic data items. The data separation means 32 transmits the reflected ultrasonic data to the ultrasonic image reconstruction means 33 and transmits the first and second photoacoustic data items to a complexification means 24. The generation of a photoacoustic image based on the first and second photoacoustic data items is the same as that in the first embodiment. The data separation means 32 inputs the separated sampling data of the reflected ultrasonic waves to the ultrasonic image reconstruction means 33.

The ultrasonic image reconstruction means 33 generates data for each line of the ultrasonic image based on the (sampling data of) reflected ultrasonic waves detected by a plurality of ultrasonic oscillators of the probe 11. For example, the ultrasonic image reconstruction means 33 adds data from 64 ultrasonic oscillators of the probe 11 at a delay time corresponding to the position of the ultrasonic oscillators to generate data for one line (delay addition method).

The detection and log conversion means 34 calculates an envelope of the data for each line which is output from the ultrasonic image reconstruction means 33 and performs log conversion for the calculated envelope. The ultrasonic image construction means 35 generates an ultrasonic image based on the data for each line subjected to the log conversion. The ultrasonic image reconstruction means 33, the detection and log conversion means 34, and the ultrasonic image construction means 35 form ultrasonic image generation means for generating an ultrasonic image based on the reflected ultrasonic waves.

The image composition means 36 composes the photoacoustic image and the ultrasonic image. For example, the image composition means 36 superimposes the photoacoustic image and the ultrasonic image to perform image composition. At that time, it is preferable that the image composition means 36 position the photoacoustic image and the ultrasonic image such that corresponding points are disposed at the same position. The composite image is displayed on image display means 14. The photoacoustic image and the ultrasonic image may be displayed on the image display means 14 side by side, without being composed, or the photoacoustic image and the ultrasonic image may be switched and displayed.

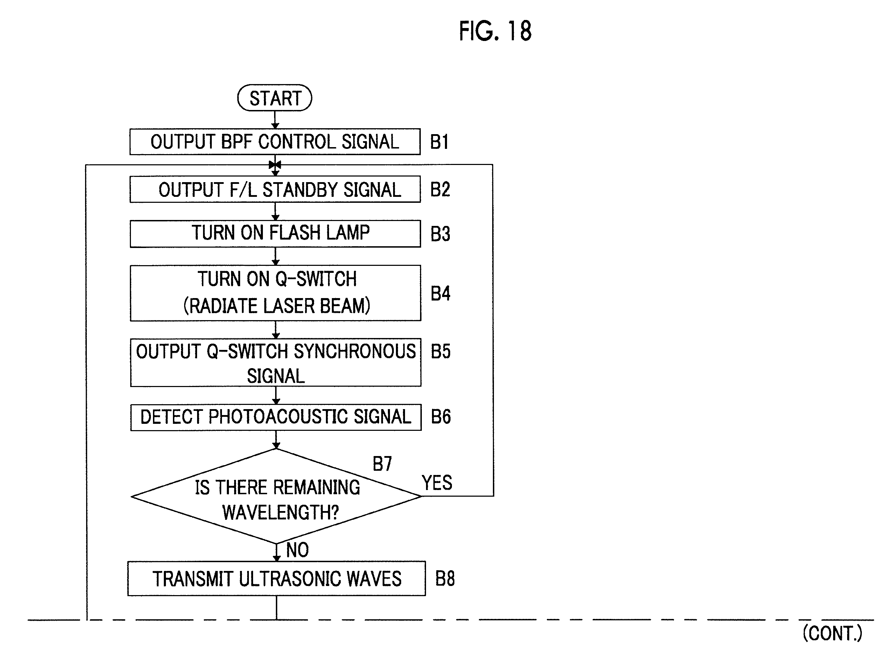

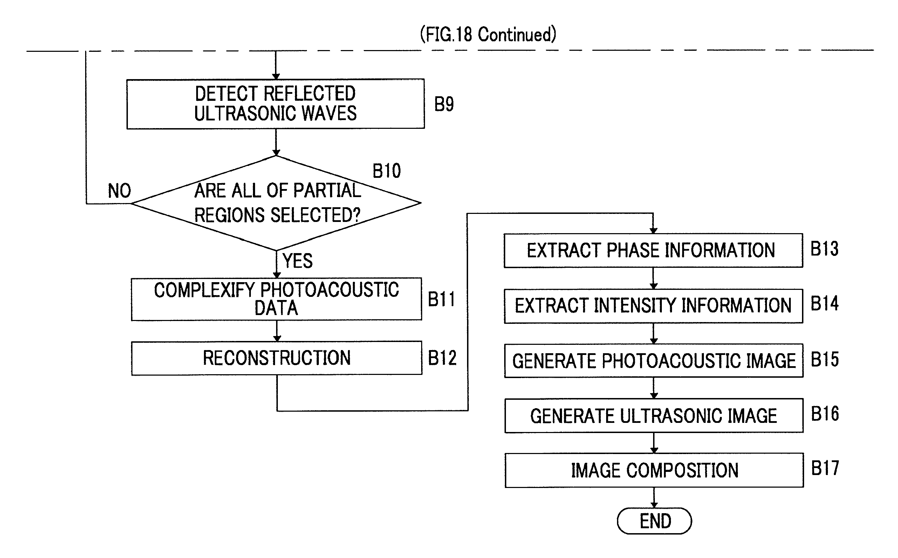

Fig. 18 shows the operational procedure of the photoacoustic image generation apparatus 10a. In the following description, it is assumed that a region of the subject which is irradiated with laser beams is divided into a plurality of partial regions. The trigger control circuit 30 outputs a BPF control signal for rotating wavelength selection means (filter rotating body) 56 of a laser source unit 13 at a predetermined rotational speed to the laser source unit 13, prior to the radiation of the pulsed laser beam to the subject (Step B1).

When the trigger control circuit 30 is ready to receive a photoacoustic signal, it outputs a flash lamp standby signal to the laser source unit 13 at a predetermined time in order to radiate a pulsed laser beam with a first wavelength (for example, 750 nm) (Step B2). After receiving the flash lamp standby signal, a light emission control unit 61 of the laser source unit 13 transmits a flash lamp control signal to a flash lamp 52 to turn on the flash lamp 52 (Step B3). The light emission control unit 61 outputs the flash lamp control signal based on BPF state information, for example, at the time that is calculated back from the time when the rotational displacement position of the wavelength selection means 56 is switched from a non-transparent region 74 (Fig. 3) to a first transparent region 71 which transmits light with a wavelength of 750 nm. When the flash lamp 52 is turned on, the excitation of a laser rod 51 starts.

After the flash lamp is turned on, the wavelength selection means 56 is continuously rotated. When a portion which is inserted onto the optical path of the optical resonator is switched from the non-transparent region 74 to the first transparent region 71, the insertion loss of the optical resonator is rapidly changed from a large loss (low Q factor) to a small loss (high Q factor) and Q switch pulse oscillation occurs (Step B4). In this case, since the first transparent region 71 selectively transmits light with a wavelength of 750 nm, the laser source unit 13 emits a pulsed laser beam with a wavelength of 750 nm. The light emission control unit 61 outputs, to the ultrasonic unit 12a, a Q switch synchronous signal indicating the time when a Q switch is turned on, that is, the time when the pulsed laser beam is radiated (Step B5).

The pulsed laser beam with a wavelength of 750 nm which is emitted from the laser source unit 13 is guided to, for example, the probe 11 and is radiated from the probe 11 to the first partial region of the subject. In the subject, a light absorber absorbs the energy of the radiated pulsed laser beam and a photoacoustic signal is generated. The probe 11 detects the photoacoustic signal generated from the subject. When receiving the Q switch synchronous signal, the trigger control circuit 30 outputs a sampling trigger signal to the AD conversion means 22. The AD conversion means 22 receives the photoacoustic signal detected by the probe 11 through the receiving circuit 21 and samples the photoacoustic signal with a predetermined sampling period (Step B6). The photoacoustic signal sampled by the AD conversion means 22 is stored as first photoacoustic data in the reception memory 23.

A control means 31 determines whether there is a remaining wavelength, that is, whether all of the pulsed laser beams with a plurality of wavelengths to be emitted are emitted (Step B7). When there is a remaining wavelength, the process returns to Step B2 in order to emit a pulsed laser beam with the next wavelength and the trigger control circuit 30 outputs the flash lamp standby signal to the laser source unit 13. The light emission control unit 61 transmits the flash lamp control signal to the flash lamp 52 to turn on the flash lamp 52 in Step B3. After the flash lamp is turned on, the portion which is inserted onto the optical path of the optical resonator is switched from the non-transparent region 73 to the second transparent region 72 corresponding to the second wavelength (800 nm) in Step B4 and Q switch pulse oscillation occurs. Then, the pulsed laser beam with a wavelength of 800 nm is radiated. The light emission control unit 61 outputs the Q switch synchronous signal to the ultrasonic unit 12a in Step B5.

The pulsed laser beam with a wavelength of 800 nm which is emitted from the laser source unit 13 is guided to, for example, the probe 11 and is radiated from the probe 11 to the first partial region of the subject. A light absorber in the subject absorbs the pulsed laser beam with a wavelength of 800 nm and a photoacoustic signal is generated. The probe 11 detects the generated photoacoustic signal. When receiving the Q switch synchronous signal, the trigger control circuit 30 outputs the sampling trigger signal to the AD conversion means 22. The AD conversion means 22 samples the photoacoustic signal in Step B6. The photoacoustic signal sampled by the AD conversion means 22 is stored as second photoacoustic data in the reception memory 23. The photoacoustic image generation apparatus 10a performs Steps B1 to B6 for each of the wavelengths of the pulsed laser beams to be radiated to the subject, radiates the pulsed laser beams with each wavelength to the subject, and detects the photoacoustic signal from the subject. Steps B1 to B6 may be the same as Steps A1 to A6 shown in Fig. 8.

When it is determined in Step B7 that there is no remaining wavelength, the control means 31 proceeds to a process of transmitting and receiving the ultrasonic waves. The trigger control circuit 30 directs the transmission control circuit 37 to transmit ultrasonic waves from the probe 11 to the subject (Step B8). In Step B8, ultrasonic waves are transmitted to the same region as the partial region of the subject which is irradiated with the pulsed laser beams. The probe 11 detects reflected ultrasonic waves when the ultrasonic waves are transmitted to the subject (Step B9). The detected reflected ultrasonic waves are sampled by the AD conversion means 22 through the receiving circuit 21 and are then stored as reflected ultrasonic data in the reception memory 23.

The control means 31 determines whether all of the partial regions are selected (Step B10). When the partial region to be selected remains, the process returns to Step B2. The photoacoustic image generation apparatus 10a performs Steps B2 to B7 for each partial region, sequentially radiates the pulsed laser beams with each wavelength (750 nm and 800 nm) to each partial region, and stores the first photoacoustic data and the second photoacoustic data in the reception memory 23. Steps B8 and B9 are performed to store the reflected ultrasonic data in the reception memory 23. When the radiation of the pulsed laser beams, the detection of the photoacoustic signal, and the transmission and reception of the ultrasonic waves are performed for all of the partial regions, data required to generate one frame of an ultrasonic image and a photoacoustic image is obtained.

When it is determined in Step B10 that all of the partial regions are selected, the control means 31 proceeds to a process of generating an ultrasonic image and a photoacoustic image. The data separation means 32 separates the first and second photoacoustic data items from the reflected ultrasonic data. The data separation means 32 transmits the separated first and second photoacoustic data items to the complexification means 24 and transmits the reflected ultrasonic data to the ultrasonic image reconstruction means 33. The complexification means 24 generates complex data in which the first photoacoustic image data is a real part and the second photoacoustic image data is an imaginary part (Step B11). A photoacoustic image reconstruction means 25 reconstructs an image from the complex data which is complexified in Step B11 using the Fourier transform method (FTA method) (Step B12).

Phase information extraction means 26 extracts phase information from the reconstructed complex data (Step B13). Intensity information extraction means 27 extracts intensity information from the reconstructed complex data (Step B14). Detection and log conversion means 28 performs a detection and log conversion process for the intensity information extracted in Step B14. Photoacoustic image construction means 29 generates a photoacoustic image based on the phase information extracted in Step B13 and the result of the detection and log conversion process for the intensity information extracted in Step B14 (Step B 15). Steps B11 to B 15 may be the same as Steps A9 to A13 shown in Fig. 8.

The ultrasonic image reconstruction means 33 generates data for each line of the ultrasonic image using, for example, a delay addition method. The detection and log conversion means 34 calculates an envelope of the data for each line which is output from the ultrasonic image reconstruction means 33 and performs log conversion for the calculated envelope. The ultrasonic image construction means 35 generates an ultrasonic image based on the data for each line subjected to the log conversion (Step B16). The image composition means 36 composes the photoacoustic image and the ultrasonic image and displays the composite image on the image display means 14 (Step B17).

In this embodiment, the photoacoustic image generation apparatus generates the ultrasonic image in addition to the photoacoustic image. It is possible to observe a part which is difficult to image in the photoacoustic image, with reference to the ultrasonic image. The other effects are the same as those in the first embodiment.

In each of the above-described embodiments, the first photoacoustic data and the second photoacoustic data are complexified. However, the first photoacoustic data and the second photoacoustic data may be separately reconstructed, without being complexified. In addition, in the above-described embodiments, the first photoacoustic data and the second photoacoustic data are complexified and the ratio of the first photoacoustic data to the second photoacoustic data is calculated using the phase information. However, the ratio may be calculated from the intensity information of both the first photoacoustic data and the second photoacoustic data. In this case, the same effect as described above is obtained. In addition, the intensity information is generated based on signal intensity in the first reconstructed image and signal intensity in the second reconstructed image.

When the photoacoustic image is generated, the number of wavelengths of the pulsed laser beams radiated to the subject is not limited to two, but three or more pulsed laser beams may be radiated to the subject and the photoacoustic image may be generated based on photoacoustic data corresponding to each wavelength. In this case, for example, the phase information extraction means 26 may generate, as the phase information, the magnitude relationship between the relative signal intensities of the photoacoustic data corresponding to each wavelength. In addition, for example, the intensity information extraction means 27 may generate, as the intensity information, a set of the signal intensities of the photoacoustic data corresponding to each wavelength.

In this embodiment, the number of rotations of the filter rotating body increases to reduce the switching time as much as possible. Therefore, even when the beam diameter of the light is large enough not to damage the filter (> 100 µm), Q switching can be performed. However, in this case, when the flash lamp follows rotation, Q switch repetitions increases, which is not preferable in the photoacoustic effect. In this case, the light emission frequency of the flash lamp with respect to the rotational frequency of the filter rotating body may be reduced to control the Q switch repetition rate to a desired value, thereby preventing the frequency of the Q switch pulse from being too high.

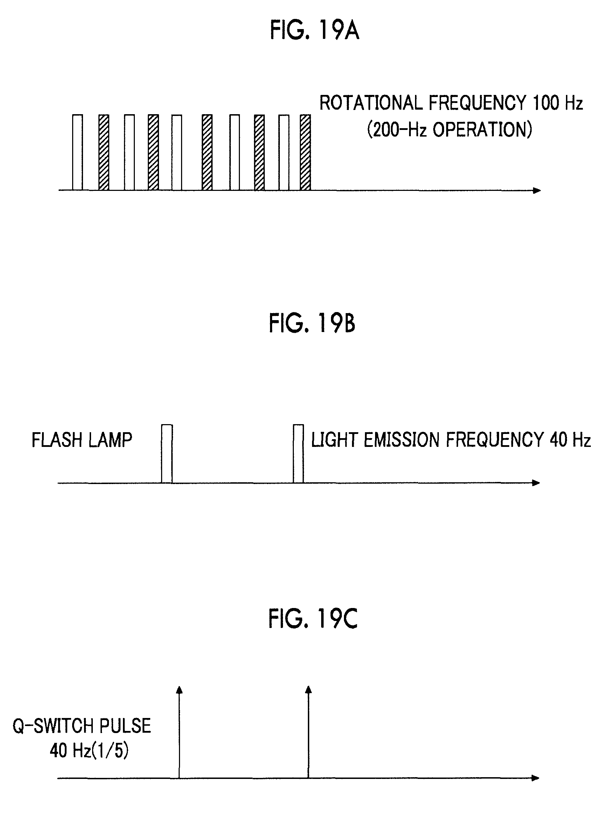

Figs. 19A to 19C show an example in which the frequency of the Q switch repetition is lower than the rotational frequency of the filter rotating body. When the wavelength selection means 56 is the filter rotating body 70 shown in Fig. 3 and the rotational frequency of the filter rotating body 70 is 100 Hz, the transparent region is inserted onto the optical path of the optical resonator at a rate of 200 times per second since the filter rotating body 70 includes two transparent regions (Fig. 19A). The light emission control unit 61 (Fig. 2) directs the flash lamp to emit light, for example, once in every five times, instead of directing the flash lamp to emit light whenever the transparent region is inserted onto the optical path of the optical resonator (Fig. 19B). In this case, it is possible to reduce the number of pulsed laser beams emitted by the laser source unit 13 per second to 40 (Fig. 19C).

As described above, it is possible to reduce the frequency of the Q switch pulse by reducing the light emission frequency of the flash lamp with respect to the rotational frequency of the filter rotating body (the number of transparent regions inserted per second). As in the above-mentioned example in which the flash lamp is directed to emit light in one in every five times, when the flash lamp emits light whenever the transparent region is inserted onto the optical path of the optical resonator, an operation is performed at 200 Hz and it is possible to achieve an operation at 40 Hz that is one fifth of 200 Hz.

For example, it is assumed that the upper limit (maximum value) of the number of times the Q switch pulse oscillation of the optical resonator can occur while the wavelength selection means 56 makes a rotation is m. For example, when the wavelength selection means 56 is the filter rotating body 80 including four transparent regions shown in Fig. 11, the upper limit of the number of times the optical resonator can perform the Q switch pulse oscillation while the wavelength selection means makes a rotation is 4 since the non-transparent region is switched to the transparent region four times per rotation in the filter rotating body 80. When the wavelength selection means is the mirror rotating body including five reflecting surfaces shown in Fig. 16, the upper limit of the number of times the optical resonator can perform the Q switch pulse oscillation while the wavelength selection means makes a rotation is 5 since the five reflecting surfaces are sequentially perpendicular with respect to the optical axis of the optical resonator while the mirror rotating body makes a rotation. When the rotational frequency when the driving means 57 rotates the wavelength selection means 56 is F [rotations/second] and n is a predetermined natural number, the light emission control unit radiates the excitation light mxF/n times per second. In this case, it is possible to reduce the frequency of the Q switch pulse to 1/n.

The exemplary embodiments of the invention have been described above. However, the laser source unit and the photoacoustic image generation apparatus according to the invention are not limited to the above-described embodiments, but various modifications and changes in the structures according to the above-described embodiments are also included in the scope of the invention.

## Claims

1. A laser source unit that emits pulsed laser beams with a plurality of different wavelengths, comprising:
a laser rod;
an excitation light source that radiates excitation light to the laser rod;
an optical resonator including a pair of mirrors that face each other with the laser rod interposed therebetween;
a wavelength selection means that controls a wavelength of light which resonates in the optical resonator to any one of the plurality of wavelengths; and
a driving means that drives the wavelength selection means such that the optical resonator performs Q switch pulse oscillation.

2. The laser source unit according to claim 1,
wherein the wavelength selection means is capable of rotary driving, and
with the rotary driving of the wavelength selection means, an insertion loss of the optical resonator changes from a first loss to a second loss which is less than the first loss.

3. The laser source unit according to claim 2, further comprising:
a light emission control unit that controls the excitation light source;
wherein the light emission control unit directs the excitation light source to radiate the excitation light at a time that is a predetermined time before a time when the wavelength selection means switches the insertion loss of the optical resonator from the first loss to the second loss.

4. The laser source unit according to claim 3,
wherein the excitation light source is turned off at the same time as the wavelength selection means switches the insertion loss of the optical resonator from the first loss to the second loss.

5. The laser source unit according to claim 3 or 4,
wherein, when an upper limit of the number of times the optical resonator performing the Q switch pulse oscillation while the wavelength selection means makes one rotation is m, a rotational frequency when the driving means rotary drives the wavelength selection means is F, and n is a predetermined natural number, the light emission control unit directs the excitation light source to radiate the excitation light for mxF/n times per second, wherein the unit of F is rotation per second.

6. The laser source unit according to any one of claims 2 to 5,
wherein a switching time when the insertion loss of the optical resonator is switched from the first loss to the second loss with the driving of the wavelength selection means is less than a generation delay time of a Q switch pulse.

7. The laser source unit according to any one of claims 1 to 6,
wherein the wavelength selection means includes a filter rotating body that includes a plurality of transparent regions and non-transparent regions which are alternately arranged along a circumferential direction, and the plurality of transparent regions selectively transmit light components with predetermined wavelengths corresponding to the plurality of wavelengths,
the driving means continuously rotates the filter rotating body such that the non-transparent regions and the transparent regions are alternately inserted onto an optical path of the optical resonator, and
when the region inserted onto the optical path of the optical resonator is switched from the non-transparent region to the transparent region, the optical resonator performs the Q switch pulse oscillation with a wavelength corresponding to the wavelength of the light which is transmitted by the switched transparent region.

8. The laser source unit according to claim 7,
wherein the transparent region includes a bandpass filter.

9. The laser source unit according to claim 7 or 8,
wherein the transparent region has a fan shape.

10. The laser source unit according to claim 7 or 8,
wherein the transparent region has a circular shape.

11. The laser source unit according to any one of claims 7 to 10,
wherein the filter rotating body is rotated in a plane which is inclined at a predetermined angle with respect to an optical axis of the optical resonator.

12. The laser source unit according to any one of claims I to 6,
wherein the wavelength selection means includes a mirror rotating body that includes a plurality of reflection regions and regions that do not reflect light, which are alternately arranged along a circumferential direction, and the plurality of reflection regions selectively reflect light components with predetermined wavelengths corresponding to the plurality of wavelengths,
the driving means continuously rotates the mirror rotating body such that the regions that do not reflect light and the reflection regions are alternately inserted onto an optical path of the optical resonator, and
the reflection regions of the mirror rotating body operate as one of the pair of mirrors, and when the region which is inserted onto the optical path of the optical resonator is switched from the region that does not reflect light to the reflection region, the optical resonator performs the Q switch pulse oscillation with a wavelength corresponding to the wavelength of the light which is reflected by the switched reflection region.

13. The laser source unit according to any one of claims 1 to 6,
wherein the wavelength selection means includes a mirror rotating body that includes a plurality of reflecting surfaces which function as one of the pair of mirrors, and the plurality of reflecting surfaces selectively reflect light components with predetermined wavelengths corresponding to the plurality of wavelengths, and
the driving means continuously rotates the mirror rotating body such that the reflecting surfaces which face the other of the pair of mirrors are sequentially switched, and when the reflecting surface is perpendicular with respect to an optical axis of the optical resonator, the optical resonator performs the Q switch pulse oscillation with a wavelength corresponding to the wavelength of the light which is reflected by the reflecting surface perpendicular with respect to the optical axis.

14. The laser source unit according to any one of claims 1 to 13, further comprising:
a condensing lens that is provided within the optical resonator and reduces a beam diameter of light which travels toward the wavelength selection means in the optical resonator.

15. The laser source unit according to claim 14,
wherein the condensing lens reduces the beam diameter of the light at the position of the wavelength selection means to 100 µm or less.

16. A photoacoustic image generation apparatus comprising:
a laser source unit that emits pulsed laser beams with a plurality of different wavelengths and includes a laser rod, an excitation light source that radiates excitation light to the laser rod, an optical resonator including a pair of mirrors that face each other with the laser rod interposed therebetween, a wavelength selection means that controls a wavelength of light which resonates in the optical resonator to any one of the plurality of wavelengths, and a driving means that drives the wavelength selection means such that the optical resonator performs Q switch pulse oscillation;
a detection means that detects a photoacoustic signal which is generated in a subject when the pulsed laser beams with the plurality of wavelengths are radiated to the subject and generating photoacoustic data corresponding to each wavelength;
an intensity ratio extraction means that extracts a magnitude relationship between relative signal intensities of the photoacoustic data corresponding to each wavelength; and
a photoacoustic image construction means that generates a photoacoustic image based on the extracted magnitude relationship.

17. The photoacoustic image generation apparatus according to claim 16,
wherein the wavelength selection means is capable of rotary driven to change insertion loss of the optical resonator from a first loss to a second loss which is less than the first loss, and
the photoacoustic image generation apparatus further includes:
a driving state detection means that detects a rotational position of the wavelength selection means;
a rotation control unit that controls the driving means such that the wavelength selection means is rotated at a predetermined rotational speed; and
a light emission control unit that directs the excitation light source to radiate the excitation light when the rotational position detected by the driving state detection means is in a position that is a predetermined distance shorter than a rotational position where the wavelength selection means switches the insertion loss of the optical resonator from the first loss to the second loss.

18. The photoacoustic image generation apparatus according to claim 17,
wherein the rotation control unit controls the driving means such that a variation in the rotational position detected by the driving state detection means for a predetermined time is constant.

19. The photoacoustic image generation apparatus according to claim 17 or 18,
wherein the light emission control unit generates a synchronous signal when the rotational position detected by the driving state detection means is in the rotational position where the wavelength selection means switches the insertion loss of the optical resonator from the first loss to the second loss, and
the detection means starts to detect the photoacoustic signal based on the synchronous signal.

20. The photoacoustic image generation apparatus according to any one of claims 16 to 19, further comprising:
an intensity information extraction means that generates intensity information indicating signal intensity based on the photoacoustic data corresponding to each wavelength,
wherein the photoacoustic image construction means determines a gradation value of each pixel in the photoacoustic image based on the intensity information, and determines a display color of each pixel based on the extracted magnitude relationship.

21. The photoacoustic image generation apparatus according to claim 20,
wherein the plurality of wavelengths of the pulsed laser beams emitted by the laser source unit include a first wavelength and a second wavelength,
the photoacoustic image generation apparatus further includes:
a complexification means that generates complex data in which one of first photoacoustic data which corresponds to the photoacoustic signal detected when the pulsed laser beam with the first wavelength is radiated and second photoacoustic data which corresponds to the photoacoustic signal detected when the pulsed laser beam with the second wavelength is radiated is a real part and the other photoacoustic data is an imaginary part; and
a photoacoustic image reconstruction means that generates a reconstructed image from the complex data using a Fourier transform method,
the intensity ratio extraction means extracts phase information as the magnitude relationship from the reconstructed image, and the intensity information extraction means extracts the intensity information from the reconstructed image.

22. The photoacoustic image generation apparatus according to any one of claims 16 to 21,
wherein the detection means further detects a reflected acoustic wave when an acoustic wave is transmitted to the subject and generates reflected acoustic wave data, and
the photoacoustic image generation apparatus further includes an acoustic wave image generation means that generates an acoustic wave image based on the reflected acoustic wave data.
